(19) Europäisches Patentamt
European Patent Office
Office européen des brevets

(11) EP 4 571 314 A1

(12) EUROPEAN PATENT APPLICATION
published in accordance with Art. 153(4) EPC

(43) Date of publication:
18.06.2025 Bulletin 2025/25

(51) International Patent Classification (IPC):
G01N 33/574 (2006.01)     G01N 33/58 (2006.01)
G01N 33/543 (2006.01)     C12Q 1/6886 (2018.01)

(21) Application number: 23853095.0

(22) Date of filing: 11.08.2023

(52) Cooperative Patent Classification (CPC):
C12Q 1/6886; G01N 33/543; G01N 33/574;
G01N 33/58

(86) International application number:
PCT/KR2023/011992

(87) International publication number:
WO 2024/035230 (15.02.2024 Gazette 2024/07)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 12.08.2022 KR 20220101571

(71) Applicant: Maxion Corp.
Seoul 06974 (KR)

(72) Inventors:
• PARK, Tae Jung
Ansan-si, Gyeonggi-do 15651 (KR)
• HUR, Cheol-Goo
Seoul 03306 (KR)

(74) Representative: Kraus & Lederer PartGmbB
Thomas-Wimmer-Ring 15
80539 München (DE)

(54) **MULTIPLE BIOMARKER FOR CANCER DIAGNOSIS AND USE THEREOF**

(57) The present invention pertains to a multiple biomarker for cancer diagnosis and a use thereof and, specifically, to a composition for cancer diagnosis, comprising an agent for measuring the protein or mRNA expression levels of IGFI, KLK2, PKCα, and TRPM8. The present inventors confirmed that rapid and accurate cancer diagnosis can be performed by detecting the unique marker combination of IGFI, KLK2, PKCα, and TRPM8 of the present invention in serum samples from actual cancer patients and one or more cancers can be simultaneously detected. Thus, the combination of IGFI, KLK2, PKCα, and TRPM8 is expected to allow for the rapid and accurate diagnosis of various cancers including prostate cancer.

FIG. 1a

EP 4 571 314 A1

## Description

Technical Field

The present disclosure relates to multiplex biomarkers for cancer diagnosis and uses thereof and, more specifically, to a composition for diagnosing cancer, the composition including agents that measure the expression levels of PKCα, IGFI, KLK2, and/or TRPM8 proteins or mRNAs thereof.

This application claims the benefit of and priority to Korean Patent Application No. 10-2022-0101571, filed on August 12, 2022, all disclosures in the specification and drawings of which are incorporated herein by reference.

Background Art

Vascular angiogenesis disorders, such as cancer, are among the most fatal threats to human health. In the United States alone, approximately 1.3 million new cancer cases arise annually, making it the second leading cause of death after cardiovascular diseases. Despite significant medical advances in treating specific types of cancer, the overall 5-year survival rate for all cancers has improved by only about 10% over the past 20 years. Cancer or malignant tumors metastasize and grow uncontrollably, making timely detection and treatment extremely challenging.

Globally, the importance of early diagnosis of cancer diseases has gained significant attention, leading to an increase in research on early detection methods. However, existing diagnostic techniques primarily rely on invasive methods such as tissue sampling and endoscopic examinations. Particularly, tissue biopsies involve excising suspected areas for microscopic observation. Such procedures often require invasive measures, including using needles, punches, endoscopes, or laparoscopes to penetrate the body, causing considerable discomfort for patients, leaving scars, and necessitating long recovery periods.

Prostate cancer (PCa) is among the leading causes of cancer-related deaths in men in the United States. Statistics demonstrate that in 2016, prostate cancer, respiratory cancer, and colorectal cancer collectively accounted for 46% of cancer-related deaths. Prostate cancer has also been reported to be linked to factors associated with economic development, becoming a growing issue in densely populated developing countries in Asia. In South Korea, from 2007 to 2013, the prevalence of prostate cancer tripled, and its mortality rate rose sharply, particularly among younger adults (<70 years).

Conventional prostate cancer screening methods involve measuring serum prostate-specific antigen (PSA) levels, followed by digital rectal examinations and diagnostic biopsies. Currently, prostate cancer diagnosis relies on physician discretion if the serum PSA level exceeds 4 ng/mL. While these methods can detect most malignant tumors, concerns about the efficacy of standard PSA testing have recently emerged. Only about 25% of men with elevated PSA levels (>4.0 ng/mL) are diagnosed with prostate cancer following biopsies, and false negatives are common. Moreover, potentially hazardous biopsies may fail to identify cancer due to tumor heterogeneity, often necessitating repeated procedures. Furthermore, the U.S. Preventive Services Task Force (USPSTF) has issued final recommendations advising against PSA-based tests for prostate cancer diagnosis.

In response, the present inventors sought to establish a novel combination of biomarkers and a diagnostic method that could complement PSA testing for enhanced prostate cancer diagnosis.

Disclosure of Invention

Technical Problem

Leading to the present disclosure, intensive and thorough research conducted by the present inventors on investigation into various markers to improve the accuracy and speed of cancer diagnosis resulted in the finding that the combination of KLK2, IGFI, and PKCα exhibited excellent diagnostic efficacy for various cancers including prostate and breast cancers.

Accordingly, an aspect of the present disclosure is to provide a cancer diagnostic composition including an agent that measures an expression level of at least one protein or mRNA selected from the group consisting of PKCα, IGFI, KLK2, and TRPM8.

Another aspect of the present disclosure is to provide a cancer diagnostic kit including the agent.

A further aspect of the present disclosure is to provide a lateral flow assay strip for cancer diagnosis, the strip including the agent.

Yet another aspect of the present disclosure is to provide an information provision method for cancer diagnosis.

However, the technical problems intended to be addressed by the present disclosure are not limited to the above-mentioned problems. Other problems not mentioned herein will be apparent to those skilled in the art from the descriptions provided below.

Solution to Problem

To achieve the above goals, the present disclosure provides a composition for diagnosing cancer, the composition including an agent that measures the expression levels of IGFI, KLK2, PKC$\alpha$, and/or TRPM8 proteins or mRNAs thereof.

The present disclosure further provides a cancer diagnostic use of the agent that measures the expression levels of IGFI, KLK2, PKC$\alpha$, and/or TRPM8 proteins or mRNAs thereof.

Additionally, the present disclosure provides a use for preparation of cancer diagnostic reagents of the agent that measures the expression levels of IGFI, KLK2, PKC$\alpha$, and/or TRPM8 proteins or mRNAs thereof.

The present disclosure also provides a cancer diagnostic kit comprising the composition.

Furthermore, the present disclosure provides an information provision method for cancer diagnosis, the method comprising the steps of:

(a) measuring an expression level of at least one protein or mRNA selected from the group consisting of PKC$\alpha$, IGFI, KLK2, and TRPM8 in a biological sample separated from a subject; and
(b) diagnosing cancer or determining cancer risk when the measured expression level of the protein or mRNA is increased compared to a control group.

Additionally, the present disclosure provides a method for cancer diagnosis, the method comprising the steps of:

(a) measuring an expression level of at least one protein or mRNA selected from the group consisting of PKC$\alpha$, IGFI, KLK2, and TRPM8 in a biological sample separated from a subject; and
(b) diagnosing cancer or determining cancer risk when the measured expression level of the protein or mRNA is increased compared to a control group.

In an embodiment of the present disclosure, the diagnostic composition or kit may allow simultaneous multiplex detection of one or more cancers, but with no limitations thereto.

In another embodiment of the present disclosure, the cancer may be selected from the group consisting of colorectal cancer, colon cancer, lung cancer, liver cancer, stomach cancer, esophageal cancer, pancreatic cancer, gallbladder cancer, kidney cancer, bladder cancer, prostate cancer, testicular cancer, cervical cancer, endometrial cancer, choriocarcinoma, ovarian cancer, breast cancer, thyroid cancer, brain cancer, head and neck cancer, malignant melanoma, lymphoma, aplastic anemia, and blood cancer, but with no limitations thereto.

In yet another embodiment of the present disclosure, the composition may analyze a sample selected from the group consisting of blood, plasma, serum, saliva, nasal fluid, sputum, synovial fluid, amniotic fluid, ascites, cervical or vaginal secretions, urine, and cerebrospinal fluid, but is not limited thereto.

In another embodiment of the present disclosure, the protein expression level may be measured using an antibody or aptamer specific to the protein, but with no limitations thereto.

In another embodiment of the present disclosure, the mRNA expression level may be measured using a probe or a primer that specifically bind to the mRNA, but with no limitations thereto.

In another embodiment of the present disclosure, the composition may further include an agent that measures the expression level of a prostate-specific antigen (PSA) protein or an mRNA thereof, but with no limitations thereto.

In another embodiment of the present disclosure, the kit may include a lateral flow assay strip, but is not limited thereto.

In another embodiment of the present disclosure, the lateral flow assay strip may include a sample pad, a conjugation pad, a membrane, and an absorbent pad, but is not limited thereto.

In another embodiment of the present disclosure, the lateral flow assay strip may include gold nanoparticles conjugated with at least one antibody selected from the group consisting of anti-PKC$\alpha$ antibody, anti-IGFI antibody, anti-KLK2 antibody, and anti-TRPM8 antibody, but is not limited thereto.

In another embodiment of the present disclosure, the protein expression level may be measured using enzyme-linked immunosorbent assay (ELISA) or lateral flow assay methods, but with no limitations thereto.

Advantageous Effects of Invention

In the present disclosure, a combination of the specific markers IGFI, KLK2, PKC$\alpha$, and/or TRPM8 was detected in serum samples from actual cancer patients and thus was confirmed to enable rapid and accurate cancer diagnosis and allow simultaneous multiplex detection of one or more cancers including prostate and breast cancers. Particularly, the lateral flow assay strip of the present disclosure, including gold nanoparticles conjugated with an antibody specific to the biomarker, enables the simultaneous detection of multiple biomarkers, resulting in more accurate cancer diagnosis compared to single biomarker detection. Therefore, the lateral flow assay strip of the present disclosure is expected to be effectively utilized for the simultaneous diagnosis of multiple cancers.

Brief Description of Drawings

FIG. 1a shows schematic diagrams of a multiplex lateral flow assay (LFA) using multiple biomarkers for the early detection of prostate cancer: (a) an LFA strip without a sample, (b) a strip showing a positive result after the application of serum and urine samples from cancer patients, and (c) a strip showing a negative result for a healthy individual's sample.

FIG. 1b illustrates the UV-Vis spectrum of gold nanoparticles at various pH levels, showing experimental results to prevent gold nanoparticle aggregation and to select the optimal pH.

FIG. 1c shows the change in absorbance of gold nanoparticles at various pH levels, confirming that pH 9 with the smallest absorbance variation is the optimal condition.

FIG. 1d demonstrates the color changes of gold nanoparticles over time at different pH levels, showing experimental results on the stability of gold nanoparticles. Results for pH 9, with minimal color changes even after 7 days, were confirmed.

FIG. 1e displays the spectra for reaction times (up to 60 minutes at 10-minute intervals) to immobilize anti-PKC$\alpha$ antibodies (top) and anti-TRPM8 antibodies (bottom) on gold nanoparticles.

FIG. 1f is a plot for the optimal reaction time to immobilize anti-PKC$\alpha$ and anti-TRPM8 antibodies on gold nanoparticles, indicating that most antibodies were immobilized within 10 to 30 minutes.

FIG. 1g presents the results of visual/colorimetric observations of the gold nanoparticle-antibody conjugates at various antibody concentrations to identify the optimal concentration for immobilization of gold nanoparticles and antibody. The optimal concentrations for each antibody are indicated with red dotted lines (anti-KLK2: 3 $\mu$g/mL, anti-IGFI: 3 $\mu$g/mL, anti-TRPM8: 2 $\mu$g/mL, and anti-PKC$\alpha$: 4 $\mu$g/mL).

FIG. 1h shows spectra of gold nanoparticle-antibody conjugates at different antibody concentration to identify optimal antibody concentrations for immobilization of gold nanoparticles and antibody, with the optimal concentrations for each antibody indicated by red rectangles.

FIG. 1i illustrates the size (top) and surface zeta potential analysis results (bottom) of the synthesized gold nanoparticles.

FIG. 1j shows the UV-Vis spectrum results of the synthesized gold nanoparticles.

FIG. 1k presents electron microscopy images of gold nanoparticles: (a) gold nanoparticles approximately 40 nm in diameter, (b) gold nanoparticles conjugated with antibodies, and (c-d) electron microscopy images of gold nanoparticles conjugated with antibodies applied to absorbent pads.

FIG. 1l shows FTIR spectra of gold nanoparticles conjugated with KLK2, IGFI, TRPM8, or PKC$\alpha$ antibodies.

FIG. 2 depicts the cell growth curves of various cell lines to verify the growth stages of cancer cells.

FIG. 3 presents the Western blot analysis results of cell lysates at four different stages of the cell growth cycle.

FIG. 4 illustrates the verification results of KLK2, IGFI, and PKC$\alpha$ biomarkers for cancer diagnosis using indirect ELISA, with absorbances (indicating biomarker levels) compared across normal samples, prostate cancer samples, and breast cancer samples.

FIG. 5 shows plasmid maps for cloning human genes (KLK2, IGFI, PKC$\alpha$, TRPM8) into the vector pET-22b(+).

FIG. 6 provides agarose gel electrophoresis results confirming the cloning of the expression vector using restriction enzymes.

FIG. 7 presents Western blot electrophoresis results of insoluble fractions of recombinant proteins.

FIG. 8 illustrates immune-western blot electrophoresis results showing expression levels at various IPTG (Isopropyl $\beta$-D-1-thiogalactopyranoside) concentrations (0, 0.25, 0.5, 0.75, and 1 mM).

FIG. 9 provides immune-western blot electrophoresis results verifying the expression levels of each biomarker at different expression temperatures (2: 12°C, 3: 25°C, and 4: 37°C).

FIGS. 10a to 10f show the results of indirect ELISA (FIGS. 10a to 10c) and sandwich ELISA (FIGS. 10d to 10f) analyses using purified PKC$\alpha$, IGFI, and KLK2 biomarker proteins for cancer diagnosis.

FIGS. 11a to 11d show the lateral flow assay results for the IGFI antigen (FIG. 11a: changes in the test/control band intensity and images of LFA strips (inset) for various IGFI antigen concentrations (1 ng/mL to 8000 ng/mL); FIG. 11b: Log-adjusted results of IGFI bands from FIG. 11a; FIG. 11c: Changes in the test/control band intensity and images of LFA strips (inset) for serum spiked with IGFI antigens at various concentrations (1 ng/mL to 2000 ng/mL); and FIG. 11d: Calibration results for various concentrations of IGFI antigen in serum spiked samples).

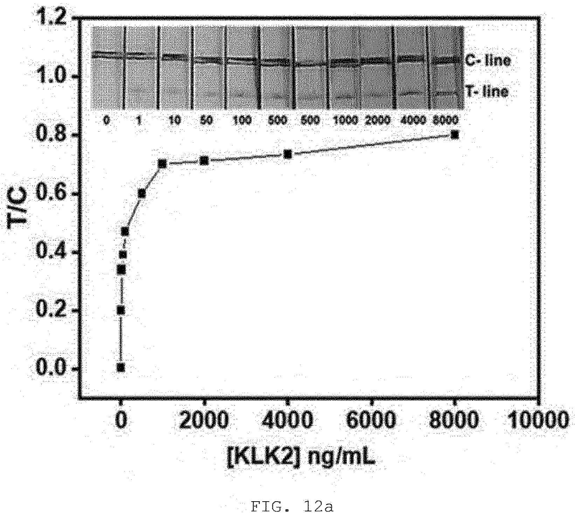

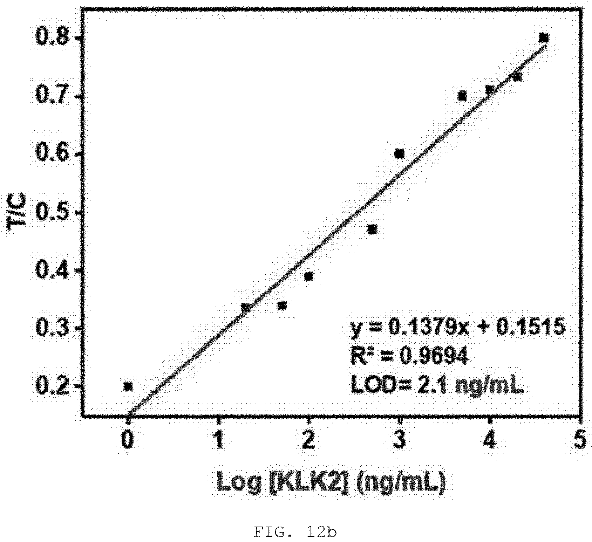

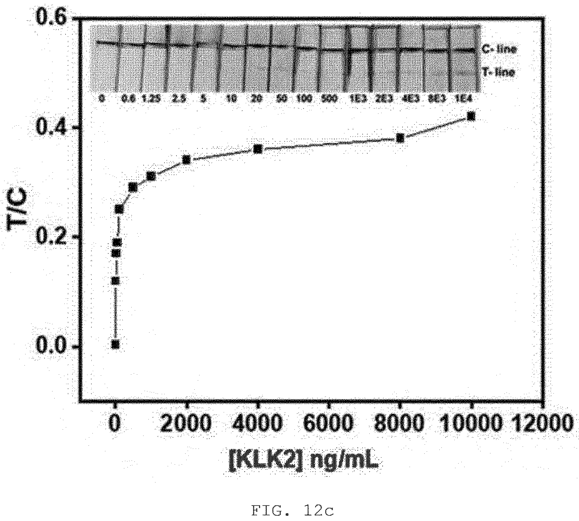

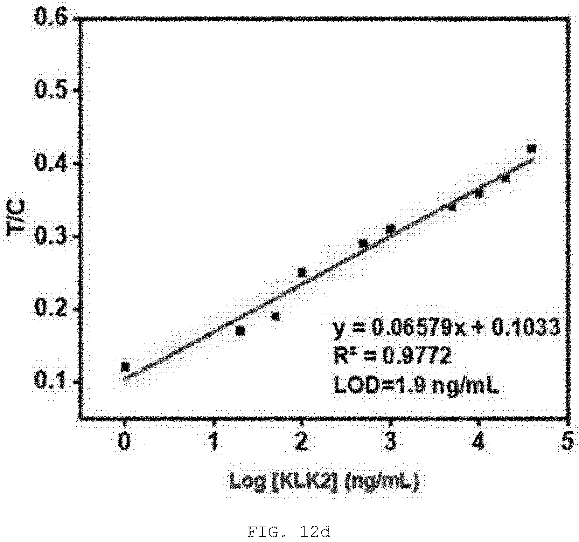

FIGS. 12a to 12d illustrate the results of lateral flow assay (LFA) for the KLK2 antigen (FIG. 12a: Changes in test/control (T/C) band intensity and corresponding images of LFA strips (inset) across various concentrations of KLK2 antigen (1 ng/mL to 8000 ng/mL); FIG. 12b: Logarithmic calibration results for the KLK2 bands shown in Figure 12a; FIG. 12c: Changes in T/C band intensity and corresponding images of LFA strips (inset) for serum-spiked samples containing the target antigen KLK2 at various concentrations (1 ng/mL to 1000 ng/mL); and FIG. 12d: Calibration results for various concentrations of serum-spiked samples containing KLK2).

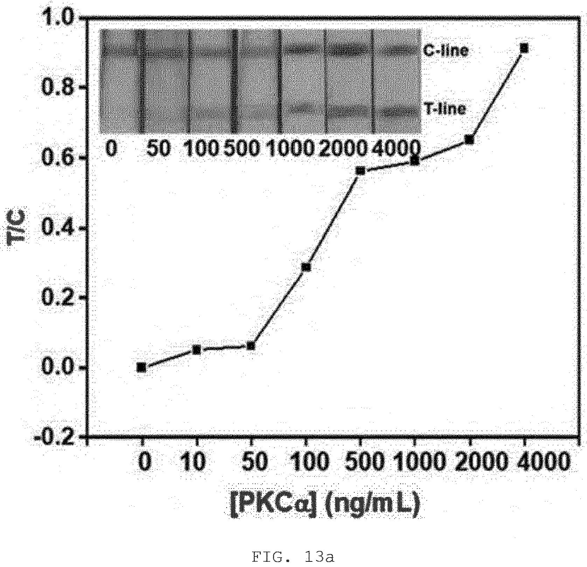

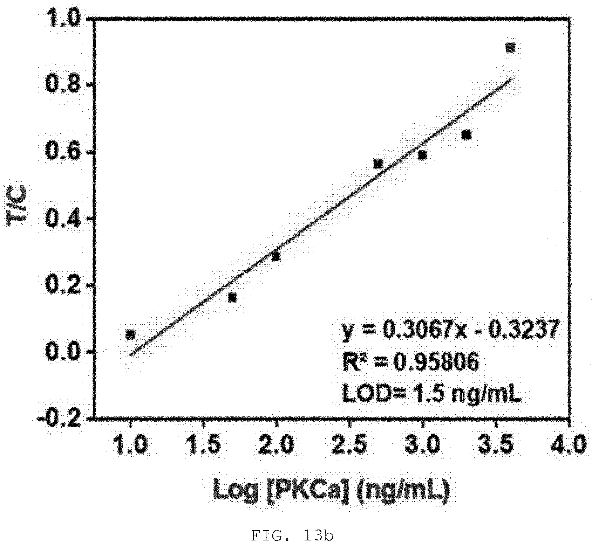

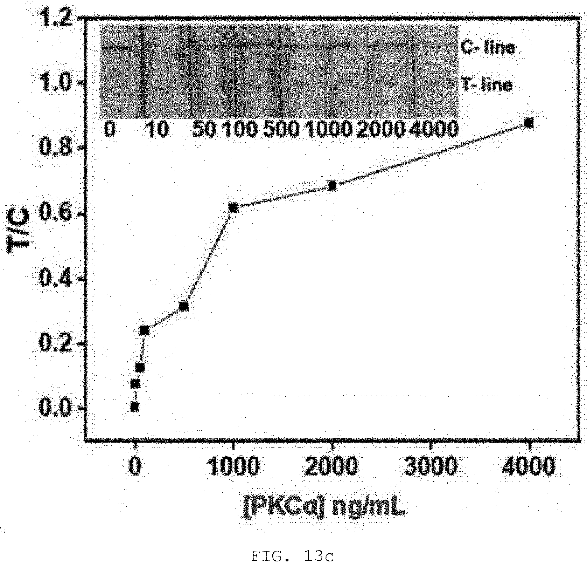

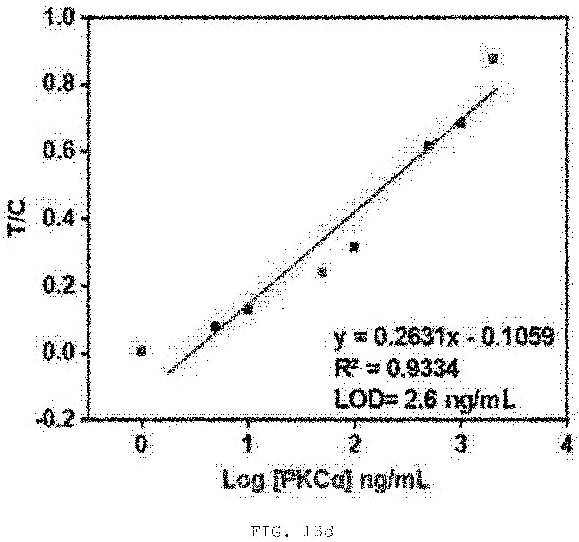

FIGS. 13a to 13d illustrate the results of LFA analysis for the PKCα antigen (FIG. 13a: Changes in T/C band intensity and corresponding images of LFA strips (inset) across various concentrations of PKCα antigen (1 ng/mL to 4000 ng/mL); FIG. 13b: Logarithmic calibration results for the PKCα bands shown in Figure 13a; FIG. 13c: Changes in T/C band intensity and corresponding images of LFA strips (inset) for serum-spiked samples containing the target antigen PKCα at various concentrations (1 ng/mL to 4000 ng/mL); and FIG. 13d: Calibration results for various concentrations of serum-spiked samples containing PKCα).

FIG. 14 shows diagnosis results for the IGFI biomarker using LFA strips with serum samples from healthy individuals and cancer patients (liver cancer, lung cancer, colorectal cancer, breast cancer, and prostate cancer).

FIG. 15 shows diagnosis results for the KLK2 biomarker using LFA strips with serum samples from healthy individuals and cancer patients (liver cancer, lung cancer, colorectal cancer, breast cancer, and prostate cancer).

FIG. 16 shows diagnosis results for the PKCα biomarker using LFA strips with serum samples from healthy individuals and cancer patients (liver cancer, lung cancer, colorectal cancer, breast cancer, and prostate cancer).

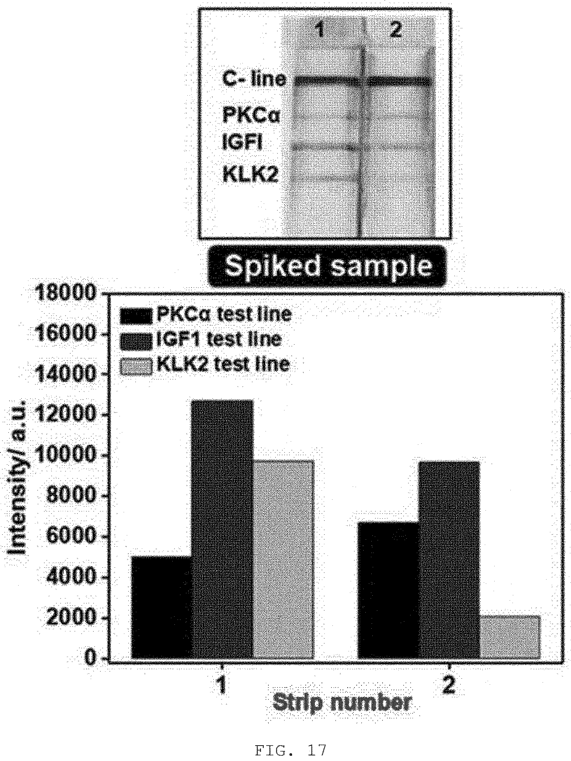

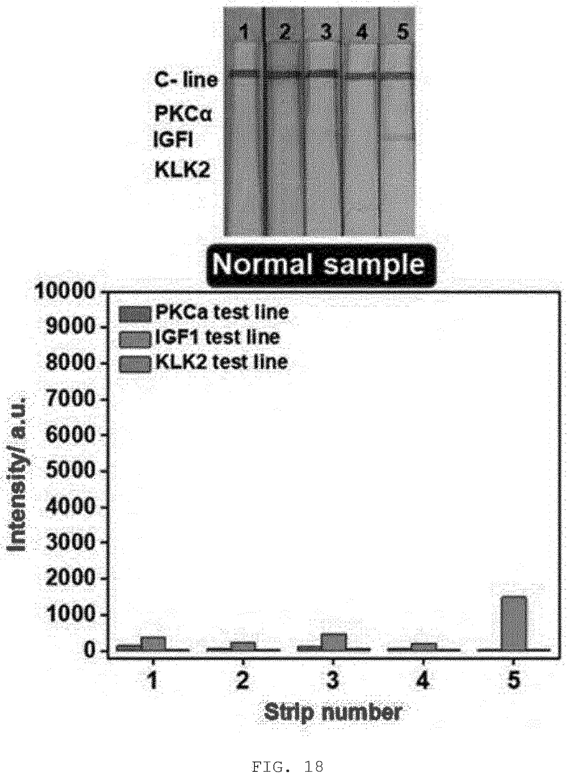

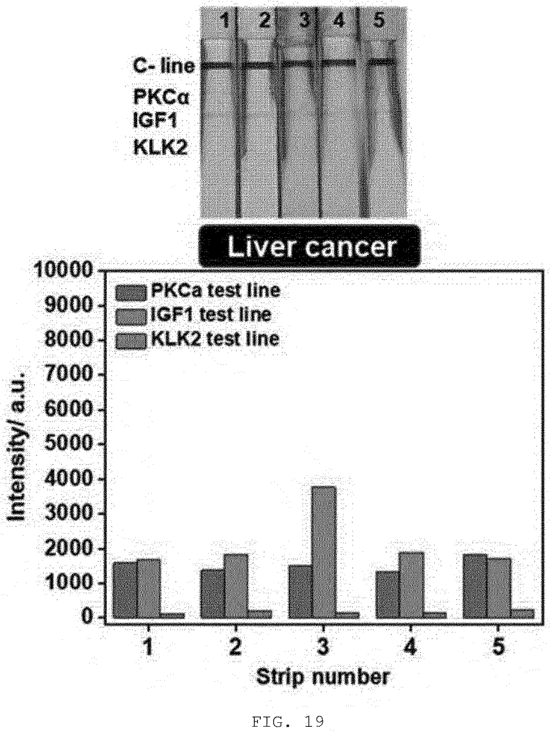

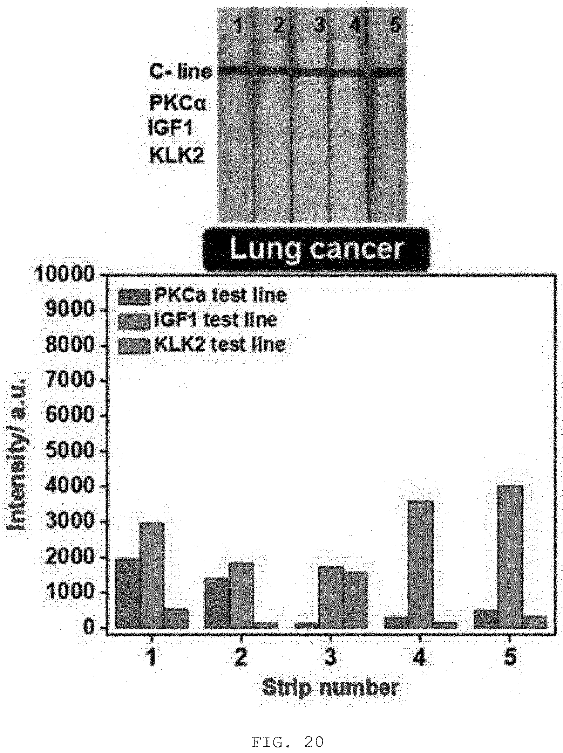

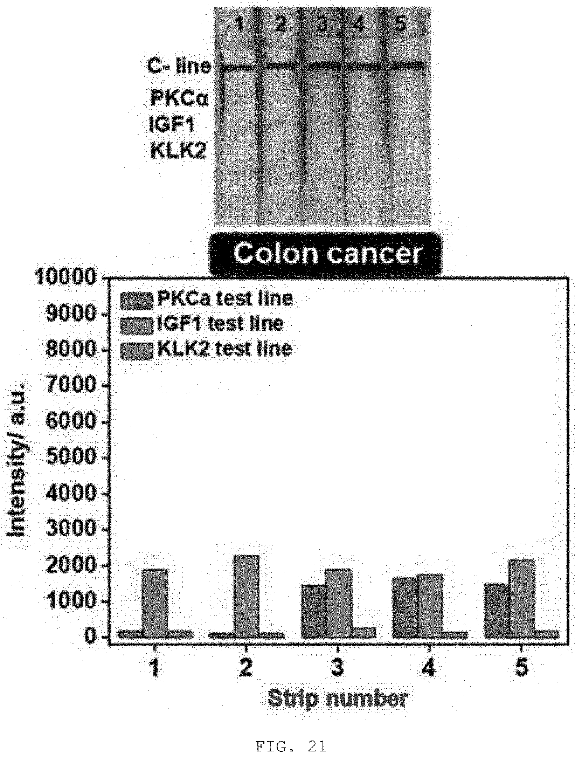

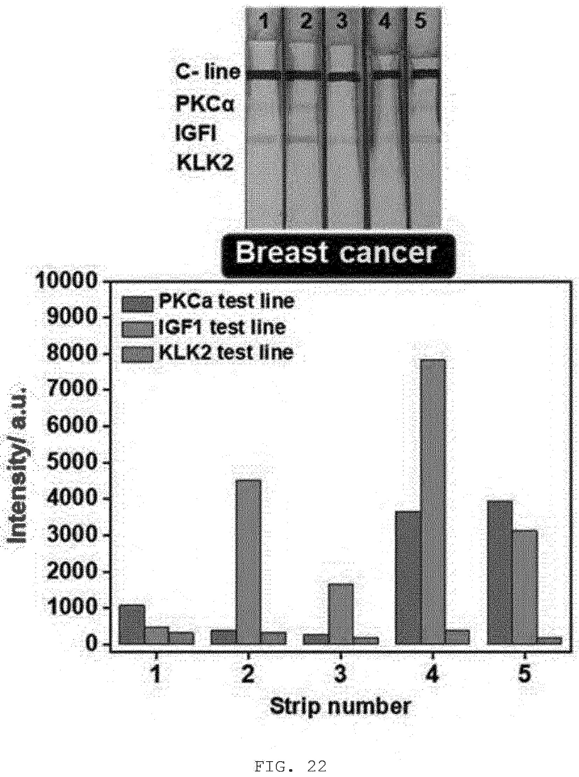

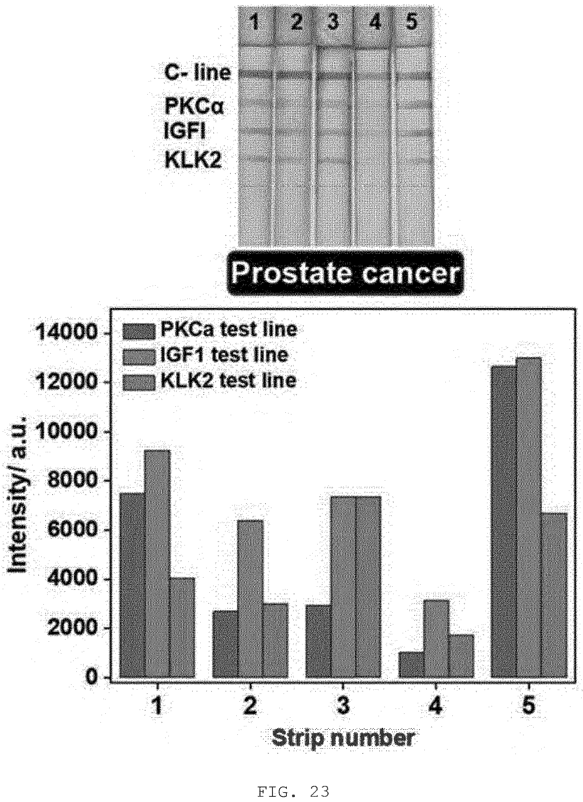

FIGS. 17 to 23 illustrate the results of simultaneous detection of PKCα, IGFI, and KLK2 biomarkers using multiplex LFA strips for various samples. Each figure contains images of the LFA results on the top and corresponding T/C band intensity graphs on the bottom (FIG. 17: Detection results for serum-spiked samples containing 500 ng/mL of each biomarker; FIG. 18: Detection results for serum samples from healthy individuals; FIG. 19: Detection results for serum samples from liver cancer patients; FIG. 20: Detection results for serum samples from lung cancer patients; FIG. 21: Detection results for serum samples from colorectal cancer patients; FIG. 22: Detection results for serum samples from breast cancer patients; FIG. 23: Detection results for serum samples from prostate cancer patients).

Best Mode for Carrying out the Invention

In an Example of the present disclosure, for use in selecting prostate cancer biomarkers, prostate cancer-related cell lines were selected, and it was found that KLK2, TMEFF2, TRPM8, and KLK4 were well expressed in the prostate cancer cell lines (see Example 1).

In another Example of the present disclosure, the utility of KLK2, IGFI, PKCα, and TRPM8 as diagnostic markers for cancer was confirmed (see Example 2).

In yet another Example of the present disclosure, the antigen recognition efficiency of anti-IGFI, anti-KLK2, and anti-PKCα antibodies was measured using indirect ELISA and sandwich ELISA methods. The results confirmed that recombinant antigen proteins KLK2, IGFI, and PKCα were effectively detected by these antibodies (see Example 4).

In a further Example of the present disclosure, a lateral flow assay (LFA) was performed on standard samples for each biomarker to confirm their utility as cancer diagnostic biomarkers. The results demonstrated that all biomarkers were detected using LFA strips, showing good linearity and reaction times under 10 minutes for KLK2, IGFI, and PKCα (see Example 6).

In yet another Example of the present disclosure, LFA strips were used to analyze clinical samples of various cancers, including breast cancer, lung cancer, liver cancer, colon cancer, and prostate cancer. The results showed that KLK2, IGFI, and PKCα had high activity in cancer patient samples, with sensitivities, specificities, and accuracies ranging from 90% to 100%, confirming their excellent diagnostic performance (see Example 7).

In another Example of the present disclosure, a multiplex LFA strip was used for the simultaneous detection of multiple biomarkers in clinical samples. When the strip was applied to cancer patient samples, the biomarkers of the present disclosure were simultaneously detected, enabling more accurate cancer diagnoses (see Example 8).

Accordingly, the present inventors completed the diagnostic composition of the present disclosure by confirming that KLK2, IGFI, PKCα, and TRPM8 can be used for accurate and rapid cancer diagnosis.

Below, a detailed description will be given of the present disclosure.

An aspect of the present disclosure pertains to a cancer diagnostic composition including an agent that measures expression levels of the proteins PKCα, IGFI, KLK2, and/or TRPM8, or mRNAs thereof. The present disclosure also provides a cancer diagnostic kit including the agent.

As used herein, the term "polynucleotide" or "nucleic acid" refers to deoxyribonucleic acid (DNA) or ribonucleic acid (RNA) in single- or double-stranded form. Unless otherwise specified, the term also includes known analogs of naturally occurring nucleotides that hybridize to natural nucleotides in a similar manner. Typically, DNA consists of four bases: adenine (A), guanine (G), cytosine (C), and thymine (T), while RNA contains uracil (U) instead of thymine. In a nucleic acid double strand, A forms hydrogen bonds with T or U, and C pairs with G through hydron bonds, and these base relationships are referred to as "complementary."

Additionally, "mRNA" (messenger RNA) refers to RNA that acts as a blueprint for polypeptide synthesis (protein translation) by transmitting genetic information encoded in the base sequence of specific genes to the ribosome during protein synthesis. Single-stranded mRNA is synthesized during transcription, with genes serving as templates.

As used herein, the term "protein" is used interchangeably with "polypeptide" or "peptide" and refers to a polymer of amino acid residues, such as those commonly found in natural proteins.

In this specification, the single-letter (three-letter) abbreviations for amino acids correspond to the following amino acids as defined by standard biochemical nomenclature: A (Ala): Alanine; C (Cys): Cysteine; D (Asp): Aspartic acid; E (Glu): Glutamic acid; F (Phe): Phenylalanine; G (Gly): Glycine; H (His): Histidine; I (Ile): Isoleucine; K (Lys): Lysine; L (Leu): Leucine; M (Met): Methionine; N (Asn): Asparagine; O (Ply): Pyrrolysine; P (Pro): Proline; Q (Gln): Glutamine; R (Arg): Arginine; S (Ser): Serine; T (Thr): Threonine; U (Sec): Selenocysteine; V (Val): Valine; W (Trp): Tryptophan; Y (Tyr): Tyrosine

The term "complementary", as used herein, means that under predetermined hybridization or annealing conditions, specifically physiological conditions (intracellular), a target moiety in a nucleic acid molecule is sufficiently complementary to be selectively hybridized to a target (for example, SNPs in the present disclosure) and one or more mismatched base sequences may be present therein. The term is intended to encompass both substantially complementary and perfectly complementary, with more preference for perfectly complementary.

In the present disclosure, the cancer may be selected from the group consisting of colorectal cancer, colon cancer, lung cancer, liver cancer, gastric cancer, esophageal cancer, pancreatic cancer, gallbladder cancer, kidney cancer, bladder cancer, prostate cancer, testicular cancer, cervical cancer, endometrial cancer, choriocarcinoma, ovarian cancer, breast cancer, thyroid cancer, brain cancer, head and neck cancer, malignant melanoma, lymphoma, aplastic anemia, and hematological cancers. Preferably, the cancers are prostate cancer, breast cancer, liver cancer, colon cancer, and/or lung cancer, but are not limited thereto.

In the present disclosure, the Insulin-like Growth Factor I (IGFI) gene includes or consists of the nucleotide sequences of NCBI Reference Sequences NM_000618, NM_001111283.3, NM_001111284.2, or NM_001111285.3. The IGFI protein includes or consists of the amino acid sequences of NCBI Reference Sequences NP 000609.1, NP 001104753.1, NP 001104754.1, or NP_001104755.1, but is not limited thereto. The designation "IGFI" is used interchangeably with "IGF1."

In the present disclosure, the kallikrein-related peptidase 2 (KLK2) gene includes or consists of the nucleotide sequences of NCBI Reference Sequences NM_001002231.3, NM_001256080.2, or NM_005551.5. The KLK2 protein includes or consists of the amino acid sequences of NCBI Reference Sequences NP_001002231.1, NP_001243009.1, or NP_005542.1, but is not limited thereto.

In the present disclosure, the PKCα (Protein Kinase C alpha, PRKCA, AAG6, PRKACA) gene includes or consists of the nucleotide sequence of NCBI Reference Sequence NM_002737.3. The PKCα protein includes or consists of the amino acid sequence of NCBI Reference Sequence NP_002728.2, but is not limited thereto.

In the present disclosure, the Transient Receptor Potential Cation Channel Subfamily M Member 8 (TRPM8) gene includes or consists of the nucleotide sequences of NCBI Reference Sequences FJ895300.1, NM_024080.5, or XM_024453133.1. The TRPM8 protein includes or consists of the amino acid sequences of NCBI Reference Sequences ACQ66098.1, NP_076985.4, or XP_024308901.1, but is not limited thereto.

As used herein, the term "primer" refers to a short single-stranded oligonucleotide that acts as the starting point for DNA synthesis. A primer specifically binds to a template polynucleotide under suitable buffer and temperature conditions. DNA polymerase extends the primer by adding nucleoside triphosphates complementary to the template DNA, synthesizing the DNA. Primers typically consist of 15 to 30 bases, with melting temperature (Tm) varying according to base composition and length. The sequence of the primer may not have to be completely complementary to a part of the base sequence of the template. It may be sufficient that the sequence of the primer has a length and complementarity suitable for the purpose of measuring the amount of mRNA by amplifying a specific section of mRNA or cDNA by synthesizing DNA. Therefore, in the present disclosure, it may be possible that a pair of primers is easily designed by referring to the nucleotide sequence of the cDNA or genomic DNA of the mRNA. The primers for the amplification reaction may correspond to a set (pair) of primers that complementarily bind to a template at one end (sense) and a template at the other end (antisense) of a specific section of the mRNA to be amplified, respectively.

As used herein, the term "probe" refers to a fragment of a polynucleotide, typically ranging from a few to several hundred base pairs, such as RNA or DNA that can specifically bind to mRNA, cDNA, DNA, etc. of a specific gene and has a length of several to several hundred base pairs. Probes are labeled to detect the presence, absence, or expression levels of target mRNA or cDNA. Conditions for the selection and hybridization of the probe may be appropriately selected according to technologies known in the industry. For example, the probe may be used in a diagnostic method to detect alleles. The diagnostic method may include detecting methods based on hybridization of nucleic acids such as the Southern blot, and the probe bound to a substrate of a DNA chip in advance may be used in the method based on a DNA chip.

In the present disclosure, primers or probes may be chemically synthesized by a phosphoramidite solid support synthesis method or other well-known methods. In addition, primers or probes may be modified in various ways by methods known in the industry, provided such modifications do not interfere with hybridization to the target polynucleotide. Examples of such modifications may include methylation, capping, substitution of one or more homologs of a natural nucleotide, and modifications between nucleotides, such as uncharged conjugates (e.g., methyl phosphonates, phosphotriesters, phosphoroamidates, carbamates, etc.) or charged conjugates (e.g., phosphorothioate, phosphorodithioate, etc.), and combinations of labeling materials using fluorescence or enzymes.

In the present disclosure, the primer or probe is not limited to a specific sequence as long as it can measure the mRNA of the target gene.

The term "aptamer", as used herein, refers to a single-stranded nucleic acid (DNA, RNA, or modified nucleic acid) that exhibits a stable tertiary structure and binds to target molecules with high affinity and specificity. Aptamers can be developed for various target molecules (e.g., proteins, sugars, dyes, DNA, metal ions, cells) using the Systematic Evolution of Ligands by Exponential Enrichment (SELEX) method. The aptamers used in the present disclosure are not limited to specific types as long as they can detect the target proteins of the present disclosure.

The term "antibody", as used herein, refers to a protein molecule that specifically binds to an antigenic site. Antibodies can be produced using conventional methods in the art, such as hybridoma techniques, recombinant DNA methods, or phage display library methods. In some embodiments, the antibodies or fragments thereof may be derived from different organisms, including humans, mice, rats, hamsters, rabbits, or camels. Examples of antibodies include monoclonal or polyclonal antibodies, immunologically active fragments, antibody heavy chains, humanized antibodies, antibody light chains, genetically engineered single-chain Fv molecules, or chimeric antibodies. The antibody fragment includes, without restriction, fragments containing the antigen-binding region of the antibody. For instance, such fragments may be selected from the group consisting of scFv, $(scFv)_2$, Fab, Fab', and $F(ab')_2$ fragments, but with no limitations thereto.

In the present disclosure, the composition may further include an agent for measuring the expression levels of prostate-specific antigen (PSA) protein or mRNA, but is not limited thereto.

The term "kit", as used herein, refers to a tool designed to diagnose cancer by including an agent for measuring the expression levels of PKCα, IGFI, KLK2, and/or TRPM8 proteins or their mRNA. The kit may also include additional components, compositions, solutions, and devices typically required for detection methods, along with materials for detecting the expression levels of the genes, mRNA, or proteins. The detection material may be applied without restriction on the number of uses, and the sequence of application is not limited, allowing simultaneous or sequential use.

In addition to the agent for measuring the biomarker protein or mRNA levels, the kit of the present disclosure may further include tools and/or reagents known in the art for use in immunological analysis.

In the present disclosure, the kit may include a lateral flow assay (LFA) strip, but is not limited thereto. Specifically, the present disclosure provides an lateral flow assay strip capable of detecting the biomarkers PKCα, IGFI, KLK2, and/or TRPM8 according to the present disclosure. The lateral flow assay may also be referred to as lateral flow immunoassay or transverse flow analysis. The lateral flow assay strip may include a sample pad, conjugation pad, membrane, and absorbent pad, but is not limited thereto. In the lateral flow assay of the present disclosure, the target protein in the sample applied to the sample pad of the lateral flow assay strip binds to gold nanoparticle-antibody conjugates fixed on the conjugation pad and flows through the membrane (detection pad) by capillary action. The gold nanoparticles act as detection indicators (probes) and generate color upon binding to the secondary antibodies fixed in the detection area, allowing the results to be visualized. Lateral flow immunoassays rely on the colorimetric change of gold nanoparticles bound to immune complexes to evaluate detection visually. To achieve precise diagnostics and detection, high-sensitivity analysis and quantifiable reading systems are required. In an embodiment of the present disclosure, gold nanoparticles are used as marker materials to label the binding of target substances and antibodies. However, this is merely a preferred example, and any type of marker material known in the art for detecting immune complexes in LFA can be included without limitation.

Therefore, the LFA strip according to the present disclosure may include a sample pad, conjugation pad, membrane, and absorbent pad. The conjugation pad may contain a conjugate of gold nanoparticles and antibodies (i.e., antibodies immobilized on gold nanoparticles). Additionally, the antibodies can be selected from the group consisting of PKCα-specific antibodies, IGFI-specific antibodies, KLK2-specific antibodies, and TRPM8-specific antibodies, but with no limitations thereto.

The total length of the LFA strip according to the present disclosure may range from 20 to 100 mm, 20 to 80 mm, 20 to 60 mm, 20 to 55 mm, 30 to 100 mm, 40 to 100 mm, 30 to 80 mm, or 30 to 60 mm, but is not limited thereto. Additionally, the LFA strip may range in width from 1 to 20 mm, 1 to 15 mm, 1 to 10 mm, or 1 to 5 mm, but with no limitations thereto.

Preferably, the diameter of the gold nanoparticles may range from 10 to 100 nm, 10 to 80 nm, 10 to 60 nm, 10 to 50 nm, 20 to 50 nm, or 30 to 50 nm, but is not limited thereto. Furthermore, the surface charge of the gold nanoparticles may range from -100 to 0 mV, -80 to 0 mV, -60 to 0 mV, -80 to -10 mV, -80 to -20 mV, or -60 to -20 mV, but is not limited thereto.

In another embodiment of the present disclosure, the kit may be an RT-PCR kit or a microarray chip kit, but is not limited thereto.

The RT-PCR kit may include primer pairs capable of amplifying nucleic acids containing the SNP region, as well as test tubes or other suitable containers, reaction buffers, deoxynucleotides (dNTPs), Taq polymerase, reverse transcriptase enzymes, DNase, RNase inhibitors, DEPC-treated water, sterile water, and other reagents. Additionally, it may include primer pairs specific to genes used as quantitative controls.

The microarray chip kit may include a microarray comprising a substrate on which nucleic acids containing the SNP region are immobilized. Apart from including the polynucleotides, primers, or probes of the present disclosure, the microarray may otherwise be a conventional microarray. Hybridization of nucleic acids on the microarray and detection of hybridization results are well known in the art. For example, nucleic acid samples labeled with detectable markers, such as fluorescent substances like Cy3 and Cy5, can be hybridized on the microarray, and the signals emitted from the markers can be detected to determine the hybridization results.

The kit (particularly the LFA strip) according to the present disclosure is characterized by its ability to rapidly detect biomarkers and diagnose cancer accordingly. For example, the reaction time for biomarker detection using this kit may be less than 30 minutes, less than 20 minutes, less than 15 minutes, or less than 10 minutes, but is not limited thereto. The lower limit of the reaction time may be 10 seconds, 20 seconds, 30 seconds, or 1 minute. However, a person skilled in the art will understand that a lower limit within these ranges is not a mandatory requirement.

The composition and cancer are as described above. Additionally, the cancer diagnostic kit may further include one or more other components, solutions, or devices suitable for the analysis method.

In the present disclosure, the kit may include a container, instructions, and an agent for measuring the expression levels of PKC$\alpha$, IGFI, KLK2, and/or TRPM8 proteins or mRNAs thereof. The container may serve to package the material, as well as to store and secure same. The material of the container may be plastic, glass, or any other suitable material, but is not limited thereto. The instructions may include information about the characteristics and manufacturing methods of the composition or kit according to the present disclosure.

The immunological analysis described above encompasses any method capable of measuring the binding between antigens and antibodies. Such methods are well-known in the field and include, for example, immunocytochemistry, immunohistochemistry, radioimmunoassays, enzyme-linked immunosorbent assays (ELISA), immunoblotting, Farr assays, immunoprecipitation, latex agglutination, hemagglutination, nephelometry, immunodiffusion, counter-current electrophoresis, single radial immunodiffusion, protein chips, and immunofluorescence methods.

The tools and/or reagents used in immunological analysis may include suitable carriers or supports, detectable signal-generating labels, solvents, and washing agents. Additionally, when the label is an enzyme, the reagents may include substrates and reaction-stopping agents to measure enzyme activity. As mentioned above, while gold nanoparticles are used in the present disclosure as a labeling material to indicate the binding of target substances and antibodies, this is merely a preferred example. All types of labeling materials known in the art for detecting immune complexes in LFA can be included without limitation.

The antigen included in the kit of the present disclosure is preferably immobilized on a suitable carrier or support using various methods disclosed in the literature (Antibodies: A Laboratory Manual, Harlow & Lane; Cold Spring Harbor, 1988). Examples of suitable carriers or supports include agarose, cellulose, nitrocellulose, dextran, Sephadex, Sepharose, liposomes, carboxymethyl cellulose, polyacrylamide, polystyrene, granite, filter paper, ion-exchange resins, plastic films, plastic tubes, glass, polyamine-methyl vinylether-maleic anhydride copolymers, amino acid copolymers, ethylene-maleic anhydride copolymers, nylon, cups, and flat packs. Other solid substrates include cell culture plates, ELISA plates, tubes, and polymer membranes. The support can take any possible form, such as spherical (beads), cylindrical (inner surfaces of test tubes or wells), or planar (sheets, test strips).

Through specific examples, the present inventors have identified the optimal conditions for manufacturing LFA strips according to the present disclosure.

For instance, in manufacturing LFA strips, the optimal concentration of biomarker-specific antibodies for immobilization onto gold nanoparticles ranges from 1 to 10 $\mu$g/mL, 1 to 9 $\mu$g/mL, 1 to 8 $\mu$g/mL, 1 to 7 $\mu$g/mL, 1 to 6 $\mu$g/mL, 1 to 5 $\mu$g/mL, 1 to 4 $\mu$g/mL, 1 to 3 $\mu$g/mL, 1 to 2 $\mu$g/mL, 2 to 5 $\mu$g/mL, or 2 to 4 $\mu$g/mL, based on 500 $\mu$L of gold nanoparticle dispersion, but is not limited thereto.

Additionally, NaCl may be added to promote the immobilization of the antibodies onto the gold nanoparticles. The NaCl concentration may range from 5 to 30%, 5 to 20%, 5 to 15%, or 5 to 10%, with volumes ranging from 10 to 100 $\mu$L, 10 to 80 $\mu$L, 10 to 60 $\mu$L, 20 to 100 $\mu$L, 40 to 80 $\mu$L, or 40 to 60 $\mu$L, but is not limited thereto.

The immobilization of the antibodies onto the gold nanoparticles can occur within a pH range of 4 to 10, 5 to 10, 6 to 10, 7 to 10, 8 to 10, 8 to 9.5, or 8.5 to 9.5, but is not limited thereto.

Additionally, this immobilization of the antibody to gold nanoparticles can take place over 5 to 60 minutes, 5 to 50 minutes, 5 to 40 minutes, 5 to 35 minutes, or 10 to 60 minutes, but with no limitations thereto.

Another aspect of the present disclosure provides a method for providing information for cancer diagnosis or a cancer diagnostic method, the method including the steps of:

(a) measuring an expression level of at least one protein or mRNA selected from the group consisting of PKC$\alpha$, IGFI, KLK2, and TRPM8 in biological samples isolated from a subject; and

(b) diagnosing cancer or determining cancer risk if the measured expression level of the protein or mRNA is higher than that of a control group.

As used herein, the term "expression" refers to a process by which a polypeptide is produced from a structural gene. This process includes transcription of the gene into mRNA and translation of the mRNA into the polypeptide.

As used herein, the term "control group" refers to a healthy individual, but with no limitations thereto.

So long as it is known in the art, any method for detecting mRNA may be employed in the present disclosure, as exemplified by polymerase chain reaction (PCR), reverse transcription polymerase chain reaction (RT-PCR), competitive RT-PCR, real-time PCR (quantitative PCR or quantitative real-time PCR), RNase protection assay (RPA), Southern blotting, Northern blotting, or DNA chip-based methods, but with no limitations thereto.

So long as it is known in the art, any methods for measuring protein expression levels may be employed in the present disclosure, as exemplified by Western blotting, lateral flow assay (LFA), enzyme-linked immunosorbent assay (ELISA), radioimmunoassay (RIA), radioimmunodiffusion, Ouchterlony immunodiffusion, rocket immunoelectrophoresis, immunohistochemistry, immunoprecipitation assay, complement fixation assay, fluorescence-activated cell sorting (FACS), or protein chip-based methods, but with no limitations thereto. Preferably, detection may be performed using ELISA or LFA. More preferably, the diagnostic or information-providing method may be performed using the LFA strip according to the present disclosure.

ELISA includes various ELISA methods, such as direct ELISA using labeled antibodies that recognize antigens attached to a solid support; indirect ELISA using labeled secondary antibodies that recognize capture antibodies in complex of antibodies recognizing antigens immobilized to a solid support; direct sandwich ELISA using another labeled antibody that recognizes an antigen in a complex of an antibody and an antigen immobilized to a solid support, or using a labeled secondary antibody that recognizes an antibody in an antigen-antibody complex where an antibody immobilized to a solid support recognizes the antibody; or indirect sandwich ELISA using a labeled secondary antibody that recognizes the antibody after reacting the complex of an antibody and antigen immobilized to a solid support with another antibody that recognizes the antigen.

In the present disclosure, the term "subject" refers to a target for cancer diagnosis, specifically humans or non-human primates, mice, rats, dogs, cats, horses, and cows, among other mammals.

As used herein, the term "sample" refers to a biological specimen collected from a subject to analyze the presence or expression levels of PKC$\alpha$, IGFI, KLK2, and/or TRPM8 proteins or mRNA. The sample can be obtained from a subject suspected of having cancer without limitation and may include cells or tissues obtained via biopsy, blood, whole blood, serum, plasma, saliva, cerebrospinal fluid, various secretions, urine, or feces. Preferably, the sample may be selected from bronchial biopsy tissue, bronchial epithelium, nasal tissue, and lung tissue but is not limited thereto. The sample may be pretreated before use in detection, including processes such as homogenization, filtration, distillation, extraction, concentration, inactivation of interfering substances, or addition of reagents.

Additionally, the present disclosure provides a method for treating cancer, the method including the steps of:

(a) measuring an expression level of at least one protein or mRNA selected from the group consisting of PKC$\alpha$, IGFI, KLK2, and TRPM8 in a biological sample isolated from the subject;
(b) diagnosing the subject with cancer if the measured level are increased compared to a control; and
(c) treating the subject diagnosed with cancer in step (b).

As used herein, "treatment" refers to any act that ameliorates or beneficially modifies the intended disease and its associated metabolic abnormalities. Treatment methods may include chemotherapy, radiotherapy, surgery, biological therapy, or administration of antibiotics.

In the present disclosure, chemotherapy refers to the use of chemical substances for the treatment of specific diseases and encompasses all drugs used in such treatments.

Examples of the drugs include, but are not limited to, one or more anticancer agents selected from the group consisting of paclitaxel, doxorubicin, 5-fluorouracil, cisplatin, imatinib, carboplatin, oxaliplatin, tegafur, irinotecan, docetaxel, cyclophosphamide, gemcitabine, ifosfamide, mitomycin C, vincristine, etoposide, methotrexate, topotecan, tamoxifen, vinorelbine, camptothecin, daunorubicin, chlorambucil, bryostatin-1, calicheamicin, maytansine, levamisole, DNA recombinant interferon alfa-2a, mitoxantrone, nimustine, interferon alfa-2a, doxifluridine, formestane, leuprolide acetate, megestrol acetate, carmofur, teniposide, bleomycin, carmustine, heptaplatin, exemestane, anastrozole, estramustine, capecitabine, goserelin acetate, polysaccharide potassium, medroxyprogesterone acetate, epirubicin, letrozole, pirarubicin, topotecan, altretamine, toremifene citrate, BCNU, taxotere, actinomycin D, and their synthetic analogs or modified substances exhibiting equivalent pharmacological effects.

As used herein, the term "radiotherapy" refers to exposing a patient to high-energy radiation, including but not limited to X-rays, gamma rays, and neutrons. Examples of such therapies include external beam radiation therapy, internal radiation therapy, brachytherapy, proximity therapy, and systemic radiation therapy, but are not limited thereto.

As used herein, the term "surgery" is intended to encompass any therapeutic or diagnostic procedure involving methodical manual operations using the hands or instruments to achieve healing, treatment, or diagnostic effects on the body of an individual.

As used herein, the term "subject" refers to an entity requiring treatment or diagnosis of a disease. Specifically, it refers to mammals such as humans or non-human primates, mice, rats, dogs, cats, horses, and cattle.

As used herein, the term "biological therapy" refers to treatment that directly or indirectly utilizes the immune system of the human body by employing biological agents containing substances derived from organisms or produced using organisms. These biological agents include vaccines, allergens, antigens, hormones, cytokines, enzymes, blood and plasma, immune sera, monoclonal antibodies, fermentation products, antitoxins, and laboratory diagnostic agents, which cannot have their potency or stability evaluated solely through physical or chemical tests.

In the present disclosure, the biological agents may include one or more selected from the group consisting of adalimumab, alemtuzumab, bevacizumab, cetuximab, daratumumab, panitumumab, rituximab, trastuzumab, pertuzumab, ipilimumab, nivolumab, pembrolizumab, atezolizumab, durvalumab, avelumab, tocilizumab, sarilumab, satralizumab, and siltuximab, but are not limited thereto.

Throughout the specification, the term "including", "include" "comprising", or "comprise" means that other components are not excluded, but may be further included unless explicitly stated otherwise. The degree term used throughout the present invention "step of ~" or "step of the" does not mean "step for ~".

The present disclosure can undergo various modifications and have multiple embodiments. Certain embodiments are illustrated in the drawings and described in detail in the specification. However, these are not intended to limit the invention to specific forms and should be understood to include all modifications, equivalents, or substitutions within the spirit and scope of the invention. Detailed descriptions of known related technologies may be omitted if it is determined that they obscure the essence of the invention.

## Mode for Carrying out the Invention

A better understanding of the present disclosure may be obtained through the following Examples, which are set forth to illustrate, but are not to be construed to limit, the present disclosure.

## [Experimental Methods]

### 1. Reagents and Experimental Materials

Silver nitrate and phosphate-buffered saline (PBS, pH 7.4) were purchased from Sigma-Aldrich (Louis, MO, USA). Dialysis membranes (MWCO 1 kDa) were obtained from Spectrum Labs Inc. 3-(4,5-Dimethylthiazol-2-yl)-2,5-diphenyltetrazolium bromide (MTT), dimethyl sulfoxide (DMSO), and sodium phosphate monobasic monohydrate were purchased from Alpha Aesar (Ward Hill, MA, USA). Dulbecco's Modified Eagle's Medium (DMEM) and RPMI 1640 medium were purchased from GenDEPOT (Katy, TX, USA), supplemented with heat-inactivated 10% (v/v) fetal bovine serum (FBS), 100 U/mL penicillin, and 100 U/mL streptomycin. Human Hep2B liver cancer cells, HEK293 human embryonic kidney 293 non-tumor cells, RAW264.7 mouse macrophage cells, and SCC7 mouse cancer cells were obtained from American Type Culture Collection (ATCC, Manassas, VA, USA). Sodium phosphate heptahydrate was purchased from KANTO Chemical (Tokyo, Japan), and Tris base was obtained from Bio-Rad (Hercules, CA, USA). Deionized water (18.2 MΩ/cm) was filtered using a Milli-Q system (Milli-Q, Billerica, MA, USA).

### 2. Immunological Screening of Biomarkers via Cancer Cell Cultures

Prostate normal cells (RWPE1), prostate cancer cell lines (PC3, DU145, 22RV1, LNCap), and liver cancer cells (Hep3B) were purchased from ATCC (USA). The purchased cell lines were cultured in DMEM and RPMI media supplemented with 10% heat-inactivated FBS and 1% antibiotics at 37°C. The culture experiments were conducted under an atmosphere of 95% air and 5.0% $CO_2$. All cell lines were cultured in appropriate media on petri dishes and were stored on ice until lysed. Cells were washed twice with ice-cold PBS. RIPA buffer [25 mM Tris-HCl (pH 7.6), 150 mM NaCl, 1% NP-40, 1% sodium deoxycholate, 0.1% SDS] was used to lyse the cells, along with protease inhibitors (0.1 M PMSF and 10 mM sodium fluoride). The cell lysates were centrifuged at 12,000×g at 4°C for 10 minutes.

Protein concentrations were quantified using a bicinchoninic acid (BCA) kit (Sigma). Samples (50 µg) were re-suspended in SDS buffer, heated at 95°C for 5 minutes, and separated using pre-cast SDS gels (Bio-Rad). Anti-TRPM8 antibody (1:2000 dilution; Abcam #Ab109308), anti-IGFI antibody (1:2000 dilution; Abcam #Ab133542), anti-PKCα antibody (1:2000 dilution; Abcam #Ab11423), and anti-KLK2 antibody (1:400 dilution; Abcam #Ab11423) were incubated overnight on polyvinylidene fluoride (PVDF) membranes. After blocking the PVDF membranes, they were incubated overnight with anti-TRPM8 antibody, followed by washing with TBS buffer containing 0.1% Tween-20 (Abcam) and reaction with the necessary HRP-conjugated antibodies. Chemiluminescence analysis was performed using chemiluminescent substrates and a ChemiDoc Imaging System (Bio-Rad).

### 3. Expression and Purification of KLK2, IGFI, PKCα, and TRPM8 Antigens

The protein amino acid sequences of all target biomarkers were verified using the National Center for Biotechnology Information (NCBI) protein BLAST. Gene nucleotide sequences, sizes, and gene IDs corresponding to these biomarkers were reconfirmed through NCBI BLAST-n and the HUGO Gene Nomenclature Committee (HGNC). KLK2, IGFI, PKCα, and TRPM8, which are each encoded to have a C-terminal His-tag, were cloned into the pET-22b(+) vector and transformed into *Escherichia coli* BL21(DE3) for expression. The transformed *E. coli* BL21(DE3) cells containing the recombinant plasmids were inoculated into Luria-Bertani broth supplemented with 100 μg/mL ampicillin and cultured at 37°C and 25°C. When the optical density at 600 nm reached 0.35-0.4, 0.1 mM isopropyl β-D-1-thiogalactopyranoside (IPTG) was added to induce the expression of fusion proteins. After culturing at various temperatures, the cells were harvested by centrifugation at 4,254 ×g for 5 minutes, and the supernatant was discarded. The harvested cells were suspended in cell lysis buffer and lysed using ultrasonication (30 seconds on/off cycles for 10 minutes), followed by centrifugation at 15,815 ×g for 20 minutes at 4°C to obtain soluble and insoluble protein fractions (KLK2, IGFI, PKCα, TRPM8). Optimal conditions for antigen expression of KLK2 (30.6 kDa), IGFI (18.6 kDa), PKCα (76.2 kDa), and TRPM8 (123.4 kDa) were determined using polyacrylamide gel electrophoresis (SDS-PAGE). The harvested fusion proteins were purified using an AKTA purifier (GE Healthcare Bio-Science, Piscataway, NJ, USA) equipped with a Ni-column and stored at -80°C. Protein concentrations were measured using the Smart micro-BCA protein assay kit (Intron Biotechnology, Seongnam, Korea).

### 4. Preparation of Serum Samples

Serum samples from 40 cancer patients (10 each with breast cancer, colorectal cancer, and prostate cancer) and 10 healthy control samples were analyzed. Since the serum samples for use in ELISA analysis of PKA self-antibodies were not intended to differentiate between stages or locations of cancer, they were collected from patients with various active malignancies and compared to controls (normal antigen samples). The serum samples were stored at -80°C in 200 μL aliquots and thawed only once before use. Diluted serum samples were discarded after the analysis.

### 5. ELISA

Anti-ECPKA IgG autoantibodies were measured using a solid-phase sandwich ELISA. Purified human PKA antibodies (e.g., 3G2 / concentration: 1 μg/mL) were coated on the wells (100 μL) and incubated overnight at 4°C. After washing three times with washing buffer [PBST, 0.05% (w/v) Tween-20], the wells were blocked with 1% (w/v) BSA at 37°C for 2 hours. After washing twice with PBS (200 μL/well) containing 0.05% (w/v) Tween-20 and then thrice with washing buffer, 1/8,000 diluted PKA antigen (100 μL, Enzo Product) was added [dilution buffer: PBS (pH 7.4), 1% (w/v) BSA (fatty acid-free fraction V), 0.05% (w/v) Tween-20] and incubated at 37°C for 2 hours. The plate was washed and incubated with serum samples diluted at 1/20-1/30 [100 μL, dilution buffer: PBS (pH 7.4), 1% (w/v) BSA (fatty acid-free fraction V), 0.05% (w/v) Tween-20] for 2 hours at 37°C. After further washing, 1/150,000 diluted antihuman IgG-HRP conjugated antibody (100 μL) was added to PBS, 1%(w/v) BSA, 0.05%(w/v) Tween-20 and incubated at 37°C for 2 hours. After washing three times, 100 μL of TMB substrate solution was added and incubated at 37°C for 15 minutes. The reaction was stopped by adding 100 μL of 0.5 mol/L $H_2SO_4$, and absorbance was read at 450 nm using an ELISA plate reader (Synergy microplate reader, BioTek) .

### 6. Synthesis of Gold Nanoparticles and Antibody Immobilization

### 6-1. Synthesis of Gold Nanoparticles

Gold nanoparticles were synthesized using the citrate reduction method of $HAuCl_4$. Briefly, a solution of 0.01% HAuCl4 (50 mL) and 1% citrate (0.5 mL) was prepared, and the HAuCl4 solution was heated to a maximum of 95°C. Then, 0.5 mL of sodium citrate was added. Within approximately 25 seconds, the solution turned faintly blue, indicating the formation of initial gold nanoparticle nuclei. After approximately 80 seconds, the solution turned a vivid red, signifying the formation of dispersed spherical nanoparticles. The complete reduction of HAuCl4 ($Au^{3+}$ to $Au^0$) was achieved within 5-10 minutes as the initial nanoparticle nuclei formed and matured. Ten μL of the antibody solution (100 μg/mL in PBS) was added to 1.5 mL of the gold nanoparticle solution and incubated at room temperature for 30 minutes. A BSA solution (0.1% w/v, 100 μL) was then added to block the surface of the gold nanoparticles, and the mixture was incubated for 20 minutes. Subsequently, the mixture was centrifuged at 4°C, 9,358 × g for 20 minutes. The supernatant was discarded, and the pellet was resuspended in PBS containing 10% sucrose (1 mM, pH 7.4).

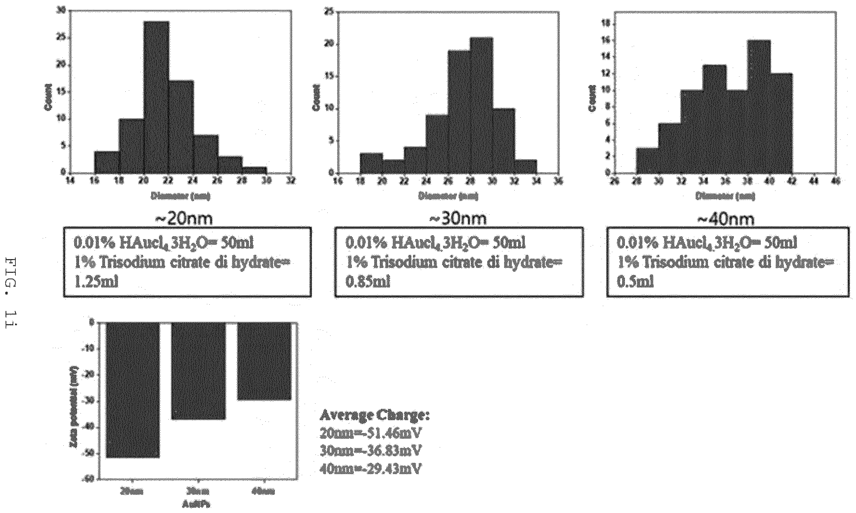

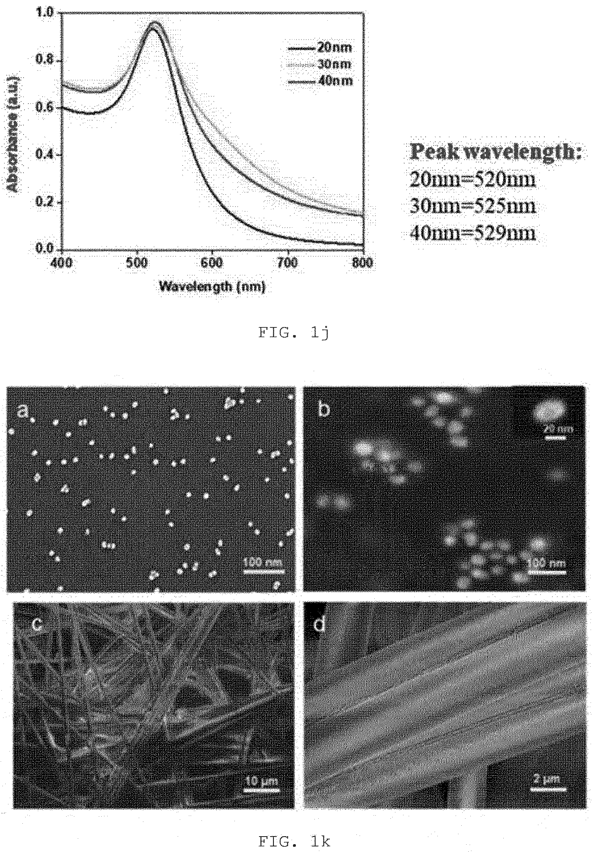

Zeta potential analysis was conducted to confirm surface charge changes of the gold nanoparticles. As shown in FIG. 1i, the surface charge decreased as the surface area increased (-51.46 mV, -36.83 mV, -29.43 mV). The absorption spectra of all gold colloid solutions, depicted in FIG. 1j, showed surface plasmon resonance peaks at 520 nm, 525 nm, and 529 nm, indicating the presence of gold nanoparticles with an increasing size distribution.

To enhance the sensitivity of the LFA biosensor, gold nanoparticles with a diameter of approximately 20-40 nm were targeted. Dynamic light scattering (DLS) analysis of the synthesized nanoparticles revealed an average diameter of 37.69 ± 3.71 nm. After antibody immobilization, the nanoparticle size increased to 45.18 ± 3.25 nm (FIG. 1k, panels (a) and (b)). Additionally, scanning electron microscopy of the antibody-functionalized gold nanoparticles applied to the LFA strip's absorbent pad confirmed the stable binding of the antibodies to the gold nanoparticles (FIG. 1k, panels (c) and (d)).

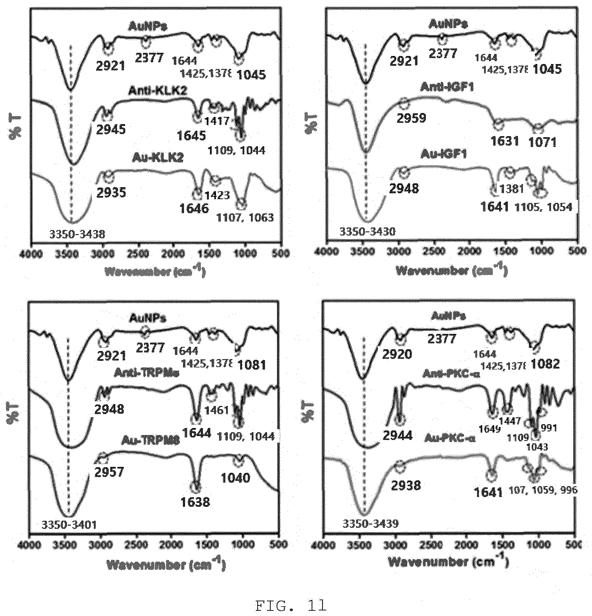

FT-IR spectroscopy was performed to verify the successful conjugation of gold nanoparticles and antibodies. The spectra confirmed the successful bond between gold nanoparticles and antibodies, specifically with the broad peak at 3350 cm$^{-1}$ corresponding to -O-H groups, and the peak at ~2950 cm$^{-1}$ corresponding to -C-H groups, indicating the presence of hydroxyl and alkane groups on the surface (FIG. 1l). The peaks at 1378 cm$^{-1}$ and 1425 cm$^{-1}$ indicated the stretching vibration of the - C=O group from citrate molecules absorbed on the nanoparticle surface. The vibration observed at ~1644 cm$^{-1}$ suggested the presence of -C=C groups on the gold nanoparticle surface. Following antibody binding, characteristic vibrations at approximately 2950, 1640, 1430, and 1380 cm$^{-1}$ corresponded to -N-H, -C-H, and -C-O groups. A bending vibration of the C-N group from the protein appeared at 1100 cm$^{-1}$, while the peak around 1050 cm$^{-1}$ implied the presence of -C-O/-C-O-C groups (FIG. 1l).

The antibodies immobilized on the gold nanoparticles used in the present disclosure are detailed in the following table, which lists.

[List of target biomarker antigens and their corresponding antibodies]

| Target Antigen | Role | Reagent | Origin | Resource |
|---|---|---|---|---|
| TRPM8 peptide | Capture | Anti-TRPM8 monoclonal antibody | Rabbit | abcam |
| | Detection | Anti-TRPM8 polyclonal antibody | Rabbit | abcam |
| Recombinant human KLK2 protein (His tag) | Capture | Anti-KLK2 monoclonal antibody [EPR6676] | Rabbit | abcam |
| | Detection | Anti-KLK2 monoclonal antibody [6B7] | Mouse | abcam |
| Recombinant human IGF1 protein (active) | Capture | Anti-IGF1 monoclonal antibody [EPR5093(2)] | Rabbit | abcam |
| | Detection | Anti-IGF1 monoclonal antibody [7973] | Mouse | abcam |
| Recombinant human PKC-α protein (active) | Capture | Anti-PKC-α monoclonal antibody [124] | Rabbit | abcam |
| | Detection | Anti-PKC-α monoclonal antibody [133] | Mouse | abcam |

## 6-2. Optimization of pH Conditions for Immobilization of Gold Nanoparticles and Antibodies

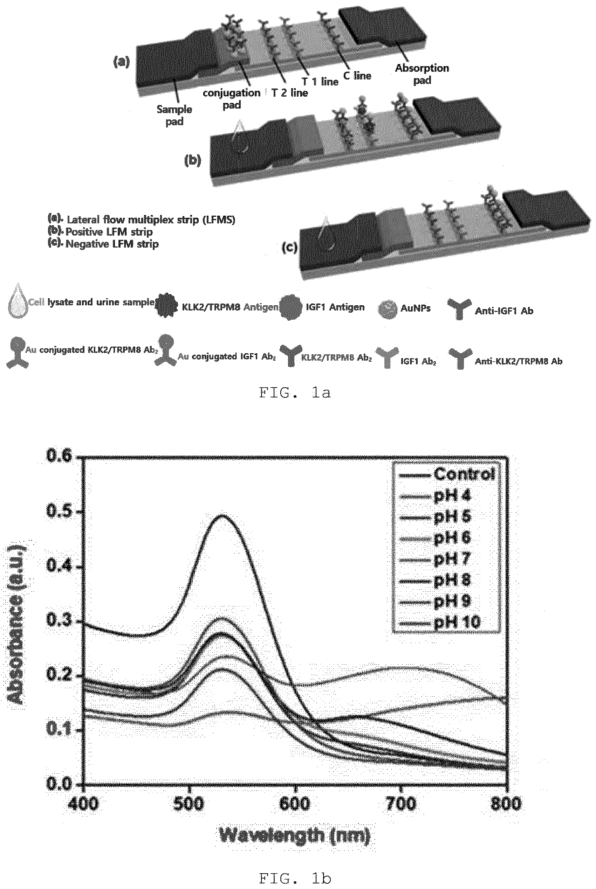

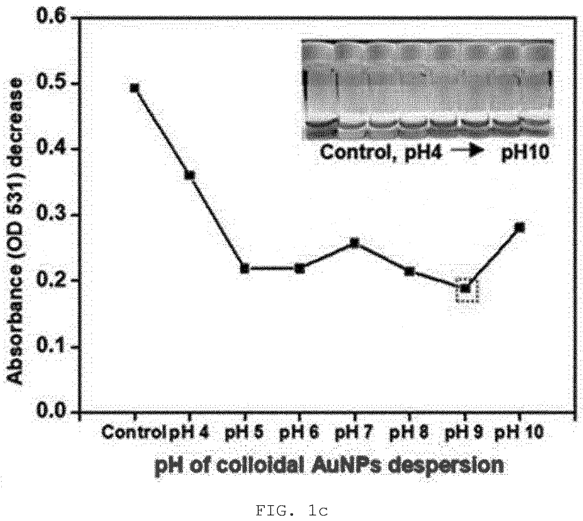

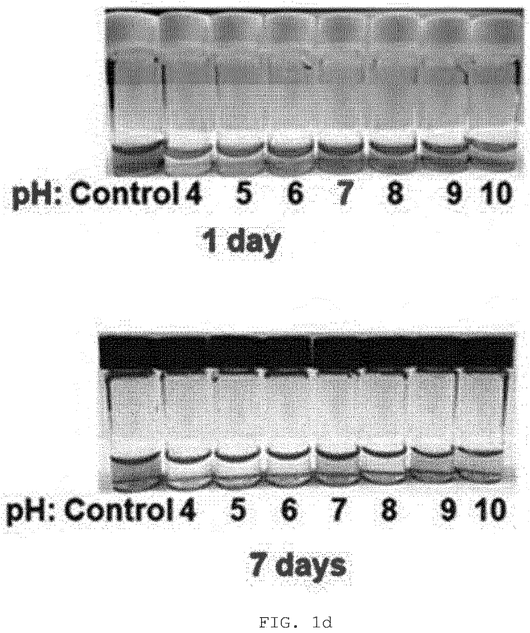

Optimization experiments were conducted at various pH conditions (4.0-10.0) to achieve effective immobilization of gold nanoparticles and antibodies. For maintaining the stability of prepared gold nanoparticles, appropriate pH conditions were required for storage and reaction. Gold colloid solutions were stable without visible precipitation at pH values above 8.0, while aggregation and precipitation occurred rapidly at pH values below 5.0. Additionally, the electrolyte concentration of the gold colloid solution was a critical factor for stabilization. Based on minimal absorbance decreases and the absence of distinct color changes (from red to blue) at pH values between 6.0 and 10.0, the optimal pH was determined to be 9.0. This conclusion was supported by UV-Vis spectra (FIG. 1b) and observable color stability (FIGS 1c and 1d, inset).

## 6-3. Optimization of Reaction Time for Immobilization of Gold Nanoparticles and Antibodies

Reaction experiments were conducted to determine the optimal concentration and time for the binding of antibodies to gold nanoparticles. The binding time varied depending on the physical properties (e.g., amino acid composition, size) and chemical properties (e.g., charge, isoelectric point [pI]) of the antibodies under the optimal pH condition.

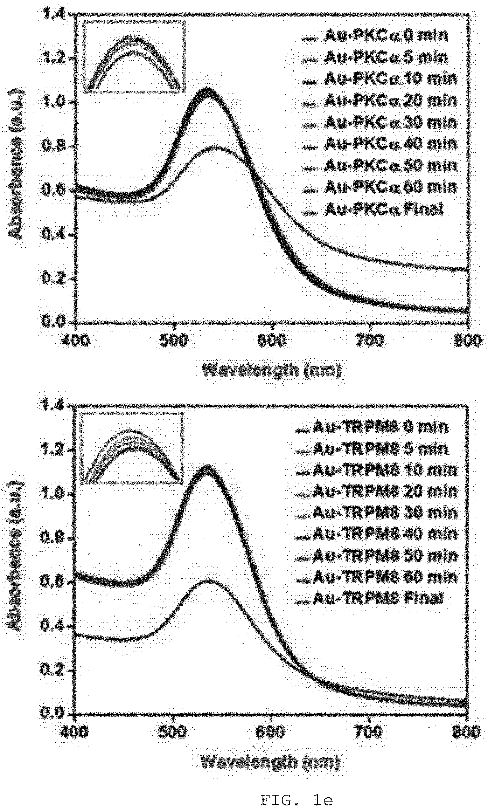

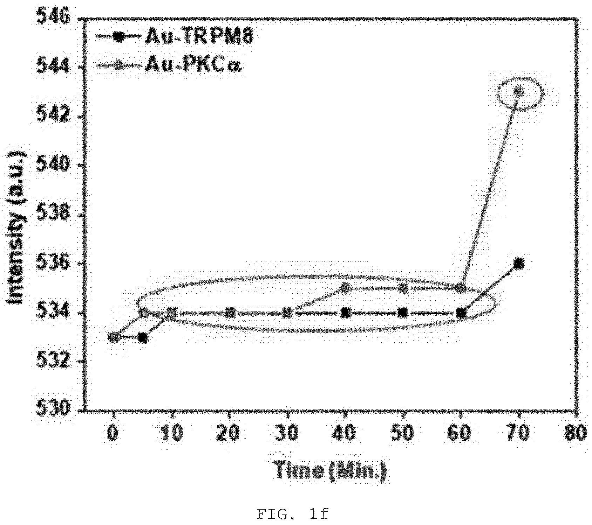

Antibody concentrations (1-20 μg/mL) were observed using a spectrophotometer by measuring spectra at 530 nm or within 400-800 nm. The degree of absorbance decrease at these wavelengths indicated the extent of binding. Anti-TRPM8 and anti-PKCα antibodies exhibited increased absorbance levels, confirming successful binding within 10 minutes and 30 minutes, respectively (FIGS. 1e and 1f). Antibodies immobilized on gold nanoparticles exhibited greater absorbance changes due to the presence of 10% (w/w) sucrose.

## 6-4. Optimization of Concentration for Immobilization of Gold Nanoparticles and Antibodies

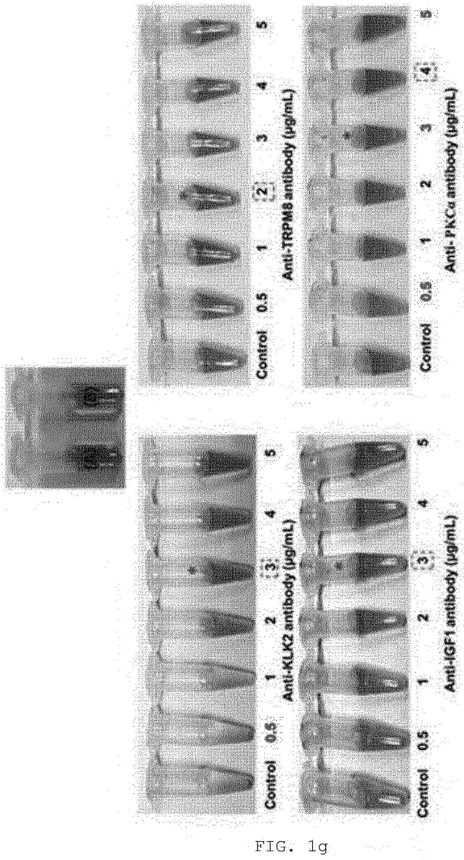

The addition of appropriate amounts of salt (NaCl) facilitated rapid and efficient aggregation of gold nanoparticles under basic pH conditions, helping determine suitable antibody concentrations for colloidal binding. After antibody binding, any unbound colloidal gold nanoparticles reacted with 10% (w/w) NaCl, inducing aggregation accompanied by color changes and optical absorbance variations. FIG. 1g illustrates colloidal gold nanoparticles (A) and aggregated gold nanoparticles (B) after salt addition.

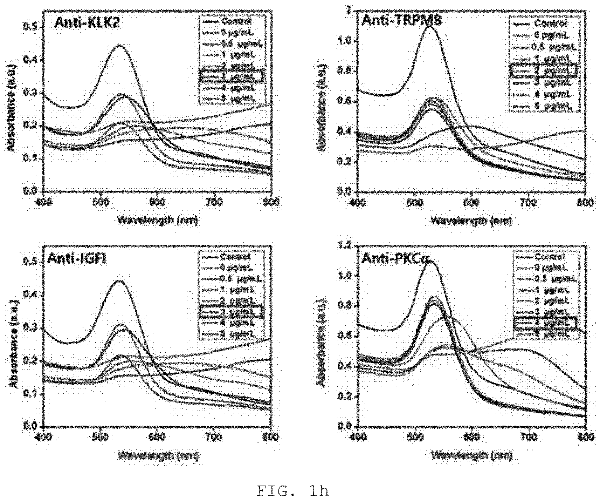

The optimal antibody concentrations for complete binding to gold nanoparticles were determined based on color changes during the reaction with colloids. The selected concentrations were as follows: 3 μg/mL for anti-KLK2, 3 μg/mL for anti-IGFI, 2 μg/mL for anti-TRPM8, and 4 ug/mL for anti-PKCα (FIG. 1g). Optimal concentrations were further confirmed through spectral changes observed during reactions with varying antibody concentrations (FIG. 1h).

The optimized conditions for immobilizing biomarker-specific antibodies onto gold nanoparticles according to the present disclosure are summarized in Tables 1 through 4.

[TABLE 1]

| Optimal Condition for Antibody Concentration for Immobilization of Anti-KLK2 Antibody to Gold Nanoparticle | | | | |
|---|---|---|---|---|
| Number | Colloidal AuNPs (μL) | Anti-KLK2 Ab (μg) | 10% NaCl (μL) | Color |
| 1 | 500 | 0 | 50 | Blue black |
| 2 | 500 | 0.5 | 50 | Blue black |
| 3 | 500 | 1 | 50 | Blue violet |
| 4 | 500 | 2 | 50 | Purplish red |
| 5 | 500 | 3* | 50 | Red |
| 6 | 500 | 4 | 50 | Red |
| 7 | 500 | 5 | 50 | Red |
| * Optimal antibody concentration | | | | |

[TABLE 2]

| Optimal Condition for Antibody Concentration for Immobilization of Anti-TRPM8 Antibody to Gold Nanoparticle | | | | |
|---|---|---|---|---|
| Number | Colloidal AuNPs (μL) | Anti-TRPM8 Ab (μg) | 10% NaCl (μL) | Color |
| 1 | 500 | 0 | 50 | Blue black |
| 2 | 500 | 0.5 | 50 | Blue black |
| 3 | 500 | 1 | 50 | Blue violet |
| 4 | 500 | 2* | 50 | Purplish red |
| 5 | 500 | 3 | 50 | Red |
| 6 | 500 | 4 | 50 | Red |
| 7 | 500 | 5 | 50 | Red |
| * Optimal antibody concentration | | | | |

[TABLE 3]

| Optimal Condition for Antibody Concentration for Immobilization of Anti-IGFI Antibody to Gold Nanoparticle | | | | |
|---|---|---|---|---|
| Number | Colloidal AuNPs ($\mu$L) | Anti-IGF1 Ab ($\mu$g) | 10% NaCl ($\mu$L) | Color |
| 1 | 500 | 0 | 50 | Blue black |
| 2 | 500 | 0.5 | 50 | Blue black |
| 3 | 500 | 1 | 50 | Blue violet |
| 4 | 500 | 2 | 50 | Purplish red |
| 5 | 500 | 3* | 50 | Red |
| 6 | 500 | 4 | 50 | Red |
| 7 | 500 | 5 | 50 | Red |
| * Optimal antibody concentration | | | | |

[TABLE 4]

| Optimal Condition for Antibody Concentration for Immobilization of Anti-PKC$\alpha$ to Gold Nanoparticle | | | | |
|---|---|---|---|---|
| Number | Colloidal AuNPs ($\mu$L) | Anti-PKC$\alpha$ Ab ($\mu$g) | 10% NaCl ($\mu$L) | Color |
| 1 | 500 | 0 | 50 | Blue black |
| 2 | 500 | 0.5 | 50 | Blue black |
| 3 | 500 | 1 | 50 | Blue violet |
| 4 | 500 | 2 | 50 | Purplish red |
| 5 | 500 | 3* | 50 | Red |
| 6 | 500 | 4 | 50 | Red |
| 7 | 500 | 5 | 50 | Red |
| * Optimal antibody concentration | | | | |

## 7. Manufacturing of Lateral Flow Strips and Biomarker Detection

The standard lateral flow assay strip comprises a sample pad, nitrocellulose (NC) membrane, absorbent pad, and adhesive backing, with the NC membrane serving as the primary component. The NC membrane's test and control zones were immobilized with KLK2 (1 mg/mL), IGFI (1 mg/mL), PKC$\alpha$ (1 mg/mL), TRPM8 (1 mg/mL), and antimouse/rabbit IgG (HRP) antibodies in a 1:10 volumetric ratio. The NC membrane was affixed to an adhesive backing, followed by sequential attachment of the sample pad and absorbent pad. As shown in FIG. 1a, the sample pad and absorbent pad were placed approximately 2 mm apart at the NC membrane's front and rear ends, respectively, enabling smooth fluid flow across the strip via optimal capillary chromatography. The assembled semi-finished strips were cut into 4 mm-wide strips and stored in the dark. In the present disclosure, various concentrations of anti-KLK2, anti-TRPM8, anti-IGFI, and anti-PKC$\alpha$ antibodies were conjugated with gold nanoparticles and reacted at 37°C for 3 hours. Subsequently, 10 $\mu$L of samples derived from cancer patients or healthy individuals were applied to the sample pad. To remove nonspecific binding on the test line, 10 $\mu$L of PBST [PBS buffer containing 0.05% (w/v) Tween-20] was allowed to flow through the entire strip. After 15 minutes, specific binding of the antigen to the antibody was confirmed. Gradual signal development was observed, as shown in FIG. 1a (b). The final strips were analyzed quantitatively by placing them in the detection channel of a strip reader. Uniform band lines appeared on either the test line or both the test and control lines in processed strips. The colorimetric intensity of the test and control lines was analyzed and recorded using a smartphone and the ChemiDoc™ MP Imaging System (Bio-Rad Laboratories).

## 8. Additional Analytical Methods

The sizes of the prepared gold nanoparticles and their conjugates with anti-KLK2, anti-IGFI, anti-PKC$\alpha$, and anti-TRPM8 antibodies were determined via dynamic light scattering (DLS) analysis. Both DLS and zeta potential measurements were performed using the Otsuka ELSZ-1000 (Otsuka Electronics, Osaka, Japan). The UV-Vis spectra were measured using a spectrophotometer (V-670, JASCO, Tokyo, Japan). The surface chemistry of gold nanoparticles and their successful conjugation with anti-KLK2, anti-IGFI, anti-PKC$\alpha$, and anti-TRPM8 antibodies were analyzed using UV-Vis spectroscopy and Fourier transform infrared (FT-IR) spectroscopy. Samples for scanning electron microscopy (SEM) analysis were attached to aluminum holders using carbon tape, then coated with a gold (Au) film at a current of 4 mA for 80 seconds before imaging.

**[EXAMPLES]**

**EXAMPLE 1: Exploration of Prostate Cancer Biomarkers**

To identify prostate cancer biomarkers, selection was made of six cell lines including four standard prostate cancer cell lines (DU145, 22RV1, PC3, and LNCaP). Additionally, normal prostate cells (RWPE1) and liver cancer cells (Hep3B) were chosen as positive and negative controls, respectively. Prostate-specific antigen (PSA) has been traditionally the most widely used biomarker for detecting prostate cancer. Unlike PSA, hK2 is known to be more strongly expressed in benign epithelial cells, particularly in malignant tumor cells. Clinical studies suggested that serum hK2 may help distinguish between malignant and benign causes of elevated PSA levels, and other prostate cancer protein biomarkers are being investigated. To replace the PSA test, further research is needed before the use of these or other potential new prostate cancer biomarkers. To be used in clinically significant cancer diagnostic methods, any of potential new prostate cancer biomarkers, although not surpass PSA, should provide complementary results for diagnosing clinically significant cancers.

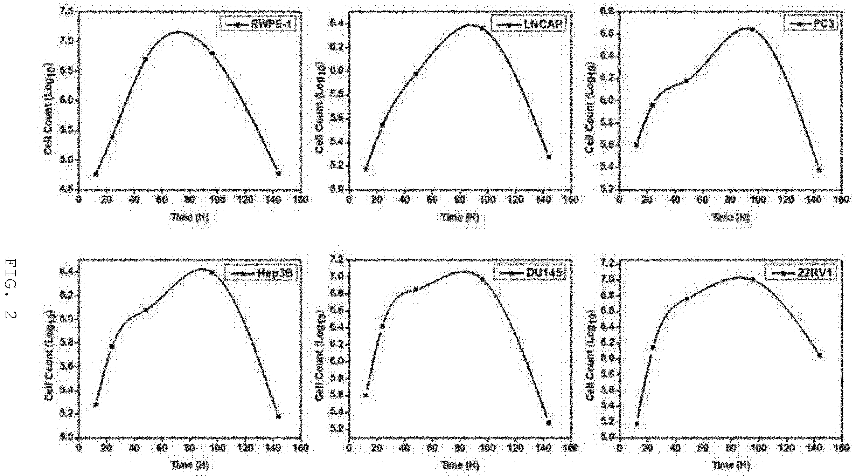

Among various known biomarkers, it is important that strong candidates such as PSP, TMEFF2, KLK4, KLK2, and TRPM8 should be considered alongside PSA to develop diagnostic methods. Cell extracts were collected using cell lysis methods. To understand the growth phases of cancer cell lines, the cell cycle stages of all selected cell lines were analyzed (FIG. 2).

The expression of all cancer biomarkers was validated through SDS-PAGE and immunological Western blot analysis. The blots confirmed the detection of proteins at their expected molecular weights. Protein expression levels were higher in cell culture supernatant sediment samples, although differences were observed among cell lines and specific proteins. Repeated experiments using culture supernatants and sediments showed inconsistent reproducibility, indicating the need to use cell extracts for accurate biomarker expression analysis.

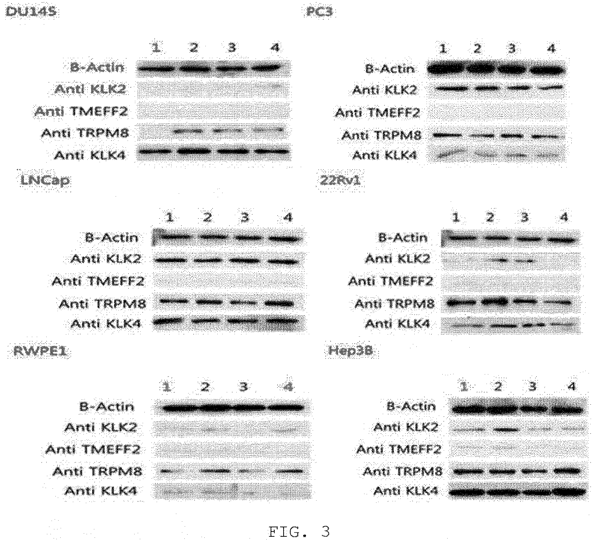

As shown in FIG. 3, the four biomarkers KLK2, TMEFF2, TRPM8, and KLK4 were detected across all four growth stages (early logarithmic phase, late logarithmic phase, stationary phase, and death phase) of the cell lines. The concentration of biomarkers varied across different cell growth phases, and expression levels were analyzed separately for each cell line at each stage. The analysis confirmed robust expression of all biomarkers based on the band sizes observed in the samples.

**EXAMPLE 2: Selection of Prostate Cancer Biomarkers**

In all experiments using cell extracts, the same number of cells was collected and analyzed for expression levels of all biomarkers. Among the biomarkers analyzed, KLK2, TRPM8, and TMEFF2 showed high and consistent expression levels, making them suitable as positive controls along with PSA. It has been reported that PSA can result in false positives in non-prostate cancer patients and false negatives in metastatic prostate cancer patients. Particularly, KLK2 and TRPM8 demonstrated strong and consistent expression across all cancer cell lines, even in the early logarithmic phase, making them especially appropriate biomarkers. Additionally, IGFI and PKC$\alpha$ were selected as supplementary biomarkers.

Insulin-like growth factor-I (IGFI) is a circulating marker useful for diagnosing and monitoring growth hormone (GH) deficiencies and excesses in clinical practice. It has also been proposed as a broader marker for estimating cancer susceptibility and non-cancerous growth characteristics (Brabant, Eur. J. Endocrinol., 148:S15-S20, 2003; Glynn and Agha, Inter. J. Endocrinol., 2012:972627, 2012). Numerous preclinical and clinical studies have linked higher circulating IGFI levels in adults to increased risks of various cancers, including breast, prostate, ovarian, endometrial, colorectal, and lung cancers, while lower risks were observed for ovarian cancer (Yu et al., J. Natl. Cancer Inst., 91:151-156, 1999; Li et al., Cell. Phys. Biochem., 38:589-597, 2016).

IGFI is known to stimulate the proliferation of normal epithelial breast cells. Studies in animal models have demonstrated a link between the IGF cascade and breast tumorigenesis, prompting extensive research into its role in breast cancer pathogenesis (LeRoith and Roberts, Cancer Lett., 195:127-137, 2003).

Chan et al. identified a significant correlation between plasma insulin-like growth factor 1 (IGFI) levels and prostate cancer risk (Chan et al., Science, 279:563-566, 1998). Multiple studies have shown that IGFI receptor signaling plays a critical role in neoplastic transformation (Pollak et al., Nat. Rev. Cancer, 4:505-518, 2004; Vella et al., Biochim. Biophys. Acta, Mol. Cell Res., 1866:118522).

The release of IGFI by epithelial cells during the progression of prostate adenocarcinoma has been reported (Wang et al., Oncogene, 38:6338-6353, 2019). Additionally, circulating IGFI levels have been linked to prostate cancer (Sreenivasulu et al., 21:138-144, 2018; Janiczek et al., J. Immunol. Res., 2020:4910595, 2020). Transgenic mouse models with direct IGFI expression in primitive prostate epithelial cells provide molecular evidence of a link between IGFI expression and prostate carcinogenesis (Ahearn et al., Carcinogenesis, 39:1431-1437, 2018).

The extensive studies conducted thus far have consistently shown that elevated IGFI levels correlate with increased prostate cancer risk. These reports support the theory that elevated serum IGFI levels in young men progress to prostate cancer after several years. Long-term exposure to high IGFI concentrations in prostate epithelial cells may induce hyperplastic cellular changes in prostate intraepithelial neoplasia, ultimately increasing the risk of prostate adenocarcinoma.

Neogenesis, persistence, and apoptosis are mediated by various signal transduction cascades and play a crucial role through phosphorylation mechanisms. Normal cells often undergo mutations into cancer cells due to altered signaling pathways that lead to the hyperactivation of kinases and subsequent molecules, ultimately enhancing unregulated proliferation or survival advantages. One of the critical groups of phosphorylation enzymes regulating these activities is known as Protein Kinase C (PKC). PKC has been extensively studied as an effector of seven transmembrane G protein-coupled receptors and tyrosine kinases. Based on biochemical and structural characteristics, PKC comprises at least 10 distinct isoenzymes categorized into three modules. These isoenzymes of are activated by diacylglycerol (DAG), a lipid second messenger generated on membranes, and by phorbol ester molecules adjacent to the DAG binding domain (C1 domain) of PKC (Kolczynska et al., Lipids Health Dis., 19:1-15, 2020; Griner and Kazanietz, Nat. Rev. Cancer, 7:281-294, 2007; Hanauske et al., Curr. Pharm. Des., 10:1923-1936, 2004). Cells express numerous PKC isoenzymes, which can distinctly overlap or even distribute biological functions across different processes.

Over the past two decades, extensive research has focused on PKC in prostate cancer (PCa) cells, particularly in LNCaP cells, which have been widely studied as a model for PKC-$\alpha$-induced apoptosis. PKC-$\alpha$ is present in most prostate cancer cell lines, with its levels being lower in hormone-responsive cell lines (e.g., LNCaP) compared to hormone-refractory lines such as PC-3, DU 145, and PC-3M (Griner and Kazanietz, Nat. Rev. Cancer, 7:281-294, 2007). PC3 prostate cancer cells exhibit elevated PKC-$\alpha$ levels, which have been linked to increased bcl-2 expression and resistance to apoptosis (Hanauske et al., Curr. Pharm. Des., 10:1923-1936, 2004). Numerous studies have identified apoptosis-inducing inhibitory treatments in LNCaP cells (Garzotto et al., Cancer Res., 59:5194-5201, 1999; Deveraux et al., EMBO J., 17:2215-2223, 1998). A key focus in this research is understanding the relative contributions of intrinsic and extrinsic apoptotic pathways to effector responses and how PKC isoforms modulate these amplification processes. The intrinsic pathway is characterized by mitochondrial depolarization, while the extrinsic pathway involves activation through death receptors. Members of the Bcl-2 receptor family are critical players in the intrinsic pathway, mediating cytochrome c release and subsequent caspase-9 activation. Conversely, the extrinsic pathway is triggered by interactions between death ligands and plasma membrane receptors, leading to recruitment of adaptor molecules and stimulation of caspase-8 and downstream caspases (Zimmermann and Green, J. Allergy Clin. Immunol., 108:S99-S103, 2001; Aggarwal, Nat. Rev. Immunol., 3:745-756, 2003). The modulation of these pathways by PKC isoenzymes is not yet fully understood. In androgen-sensitive prostate cancer cells, PKC appears to disrupt apoptotic cascades at various stages through isoform-specific mechanisms (Isakov, N. Protein kinase C (PKC) isoforms in cancer, tumor promotion and tumor suppression. in Seminars in cancer biology. 2018). Furthermore, immunoblot studies on six human colorectal cancer cell lines across different stages of tumor growth have confirmed the detection of PKC-$\alpha$ using specific antibodies in multiple cancer types (Masur et al., Mol. Biol. Cell., 12:1973-1982, 2001).

A perfect tumor biomarker could enable cancer detection via a simple blood test. However, limitations in precision and sensitivity prevent single biomarkers from being universally effective. Current serum biomarkers often focus on cancer antigen detection but lack specificity and sensitivity (Garrett and Sell, Cellular cancer markers. Vol. 12. 2013: Springer Science & Business Media). For instance, prostate specific antigen (PSA) is used as a biomarker for prostate cancer (Gretzer and Partin, Urol. Clin. North Am., 30:677-686, 2003), carcinoembryonic antigen (CEA) for colorectal cancer, CA125 for ovarian cancer (Anderiesz and Quinn, Med, J. Aust., 178:655-656, 2003), and CA19-9 for gastrointestinal cancer, and CA152 for breast cancers (Duffy et al., Tumor Biol., 40:1010428318776169, 2018). In addition to the new markers, other proteins, enzymes, and hormones have been used as indices for past 30 years (Garrett and Sell, Cellular cancer markers. Vol. 12. 2013: Springer Science & Business Media). However, these markers have very low precision and sensitivity, and their levels rise in both benign and pregnancy settings, so there is a need to improve them. Considering that the number of cancer types in which the above-mentioned various biomarkers can be detected is limited, new biomarkers are needed, and this is why the development of technology that can identify cancer types using various biomarkers is necessary.

To address this, the present disclosure leverages multiple biomarkers (KLK2, IGFI, and PKC$\alpha$) to develop more accurate diagnostic methods. Using specific IgG antibodies with binding affinity for the biomarkers, indirect ELISA analysis was conducted on diverse serum samples from actual cancer patients.

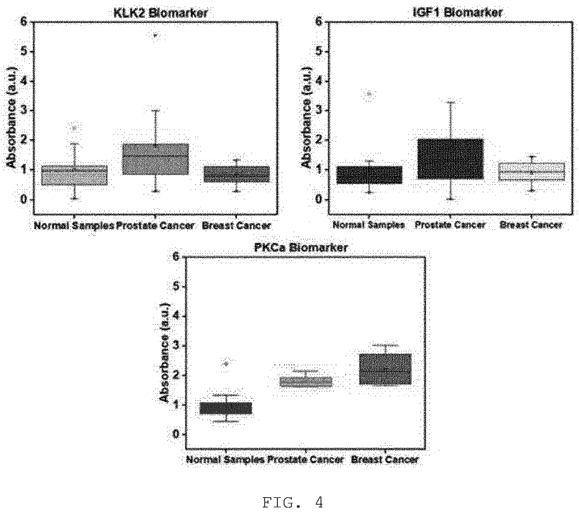

As shown in FIG. 4, KLK2, IGFI, and PKC$\alpha$ exhibited higher absorbance in samples derived from cancer patients compared to normal controls, confirming their excellent diagnostic and prognostic capabilities for breast and prostate cancer.

## EXAMPLE 3: Optimization of Expression Conditions for KLK2, IGFI, PKCα, and TRPM8

The target biomarker (TRPM8, KLK2, IGFI, PKCα) sequences were selected from prostate biomarker sequences provided by Abcam, and were acquired and verified through searches using protein database bank (PDB) and NCBI databases (NCBI BLAST and HGNC).

To obtain recombinant proteins in large quantities, human gene fragments were cloned into plasmids and expressed in *Escherichia coli.* Target vectors were transformed into *E. coli* strains Top10 and DH5α. The recombinant plasmids thus amplified were purified, and sequencing was performed to verify the accuracy of the cloned gene sequences.

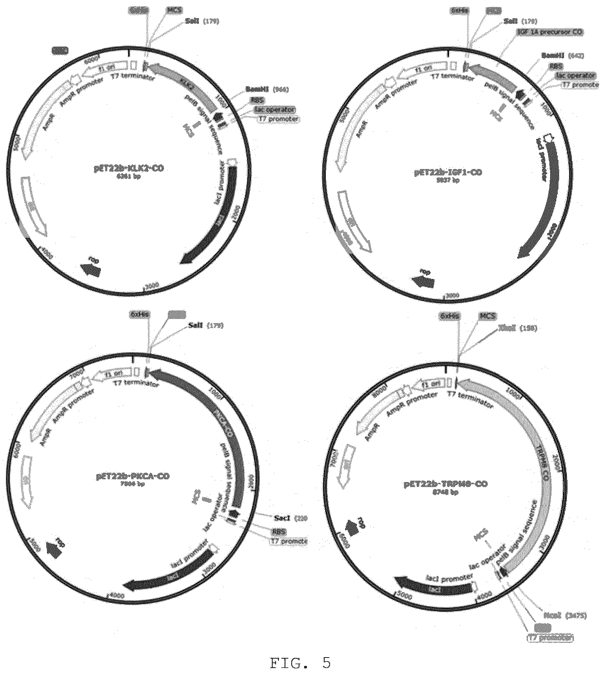

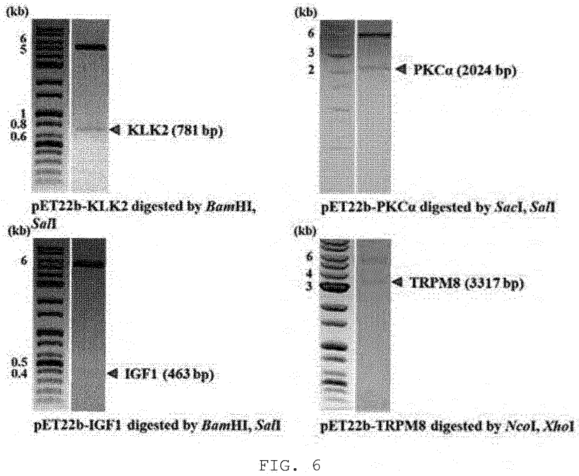

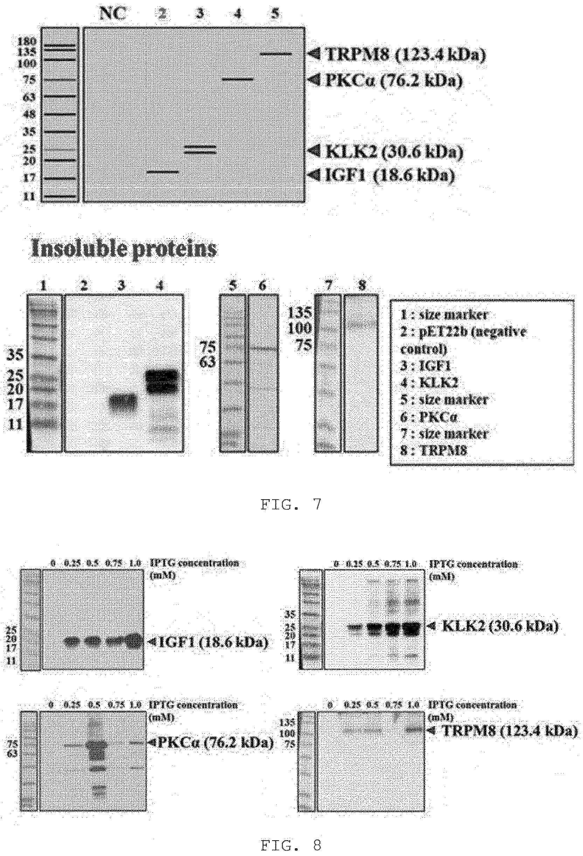

The human gene fragments verified to be successfully amplified by PCR were inserted into the pET-22b(+) expression vector for expression. The pET-22b(+) plasmid was chosen due to its advantages in terms of cost, cultivation time, and ease of handling compared to yeast and mammalian vectors. Expression plasmids (FIG. 5), including pET-KLK2, pET-IGFI, pET-PKCα, and pET-TRPM8, were transformed into *E. coli* BL21(DE3) under the control of a strong T7 promoter for recombinant protein production. Cloning was validated through restriction enzyme digestion and gel electrophoresis (FIG. 6) after digestion of the recombinant plasmids with appropriate restriction enzymes. The expression of the recombinant protein was confirmed using Coomassie staining and immunoblotting with an anti-6X His antibody (FIG. 7).

Recombinant protein expression was induced using IPTG at 37°C for six hours, showing high expression in the insoluble fraction and low expression in the soluble fraction. Recombinant protein expression was analyzed under varying IPTG concentrations (0, 0.25, 0.5, 0.75, and 1 mM) and incubation times (6 hours at 37°C, 15 hours at 25°C, and 20 hours at 12°C). Then, cells were harvested, and soluble and insoluble fractions were separated via sonication and centrifugation. Purified proteins were stored at -80°C in aliquots.

FIG. 5 illustrates a schematic of the recombinant plasmid design, showing the insertion of the recombinant genes into vectors using restriction sites (*Bam*HI, *Sal*I, *Sac*I, *Noc*I). The recombinant genes were inserted into the pelB signal sequence frame under the T7 promoter for transcription and termination by a T7 terminator sequence. A 6X His tag was attached for efficient purification.

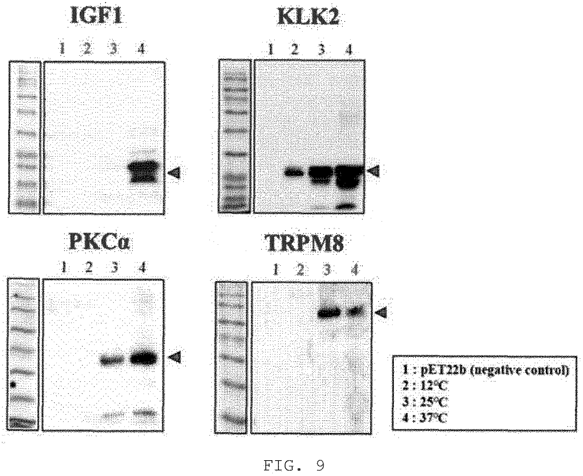

Western blot analysis of recombinant proteins showed high expression in the insoluble fraction for all proteins (FIG. 7). The insoluble fractions were found to be optimally expressed at different concentrations (0, 0.25, 0.5, 0.75, 1 mM; FIG. 8) and at different temperatures (12°C, 25°C, and 37°C; FIG. 9).

Specifically, optimal expression conditions were identified for: IGFI at 37°C, KLK2 at 12°C, 25°C, and 37°C, PKCα at 25°C and 37°C, and TRPM8 at 25°C and 37°C.

## EXAMPLE 4: Detection via ELISA

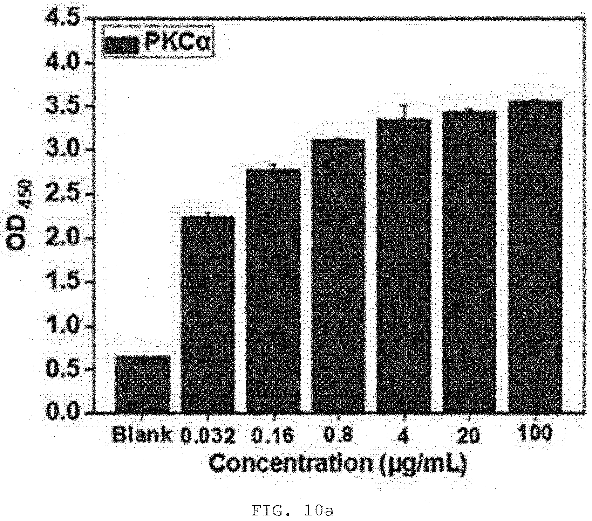

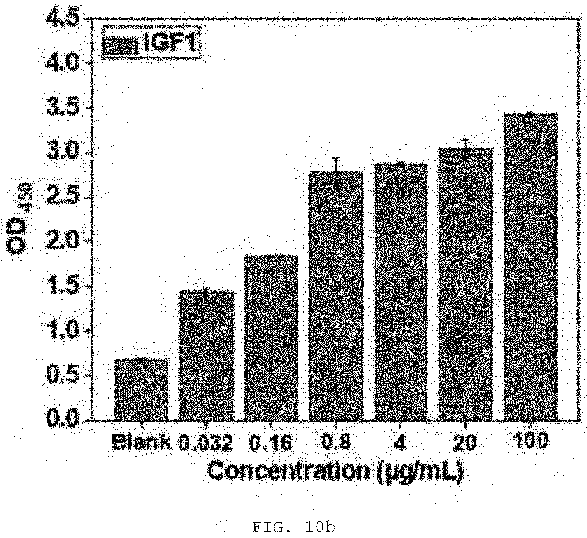

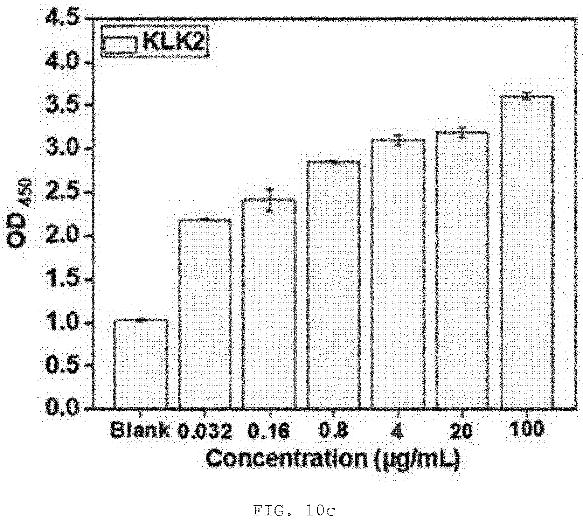

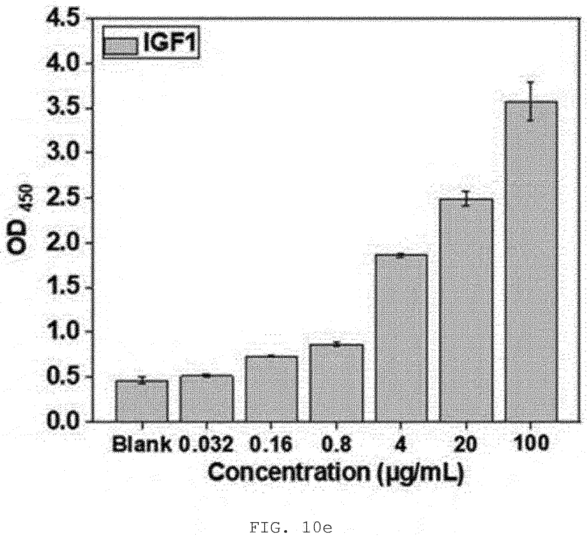

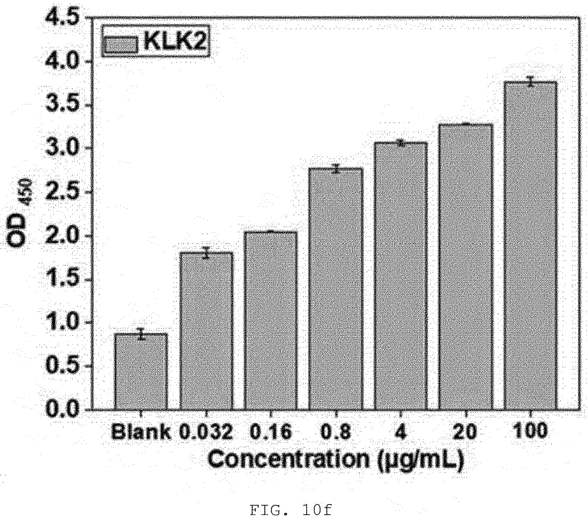

The efficiency of antigen recognition for purified IGFI, KLK2, and PKCα antigens was analyzed using indirect ELISA (FIGS. 10a to 10c).

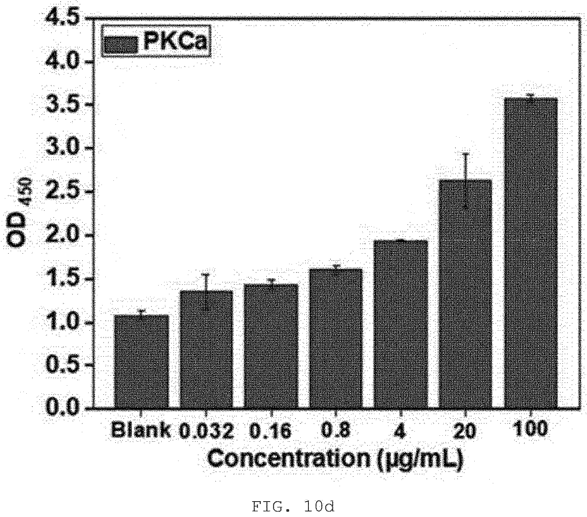

Sandwich ELISA was performed for specific and accurate detection using anti-IGFI, anti-KLK2, and anti-PKCα antibodies to analyze spiked samples containing IGFI, KLK2, and PKCα antigens (FIGS. 10d to 10f).

Recombinant antigens KLK2, IGFI, and PKCα were tested at varying concentrations (0.032 μg/mL, 0.16 μg/mL, 0.8 μg/mL, 4 μg/mL, 20 μg/mL, and 100 μg/mL) by solid-phase indirect ELISA and sandwich ELISA using respective antibodies specific therefor.

Through ELISA analyses, it was confirmed that these biomarkers, with their specific antibodies, could be utilized for the detection of biomarkers in cancer patients' serum, enabling their use for cancer diagnostics.

## EXAMPLE 5: Preparation of Lateral Flow Assay (LFA) Strips for LFA Analysis of KLK2, IGFI, PKCα, and TRPM8

To verify the utility of cancer target antigens (IGFI, KLK2, PKCα, TRPM8) as biomarkers, lateral flow assays were performed using serum samples from actual cancer patients. Gold nanoparticles were conjugated to antibodies through electrostatic interactions, formulated, and stored at 4°C for subsequent experiments. All components of the LFA strip were prepared, and both the control line (CL) and test line (TL) of the LFA strip were assembled using a dispersion system that allows solutions to flow via capillary action.

The conjugation pad was made by immersing a glass fiber membrane in a gold nanoparticle-antibody conjugate solution. The glass fiber membrane was incubated at 37°C for 2 hours for drying, then sealed with desiccant pearls in a Ziploc bag, and stored at 4°C. The sample pad was prepared by soaking a cotton membrane in PBS buffer containing 0.05% (w/v) Tween-20 and 5% (w/v) BSA. The pad was dried in an oven at 37°C for 3-4 hours and then stored in a Ziploc bag with desiccant pearls at 4°C. The detection pad was created by assembling a nitrocellulose membrane (NC membrane) onto a supporting adhesive card. Anti-KLK2, anti-IGFI, anti-PKCα, and anti-TRPM8 antibodies (1.0 mg/mL in PBS) were sprayed to form the test line (TL). Goat anti-rabbit IgG antibody (2.0 mg/mL in PBS) was sprayed 4 mm apart from the TL to form the control line (CL). The detection pad was dried at room temperature for 8 hours and stored at room temperature. All components of the strip were assembled with a 2 mm overlap to ensure smooth capillary flow. The entire strip and its components were prepared according to the dimensions specified in Table 5.

[TABLE 5]

| Sizes of Component Parts for Strip Fabrication | | |
|---|---|---|
| Sr.# | Strip components (2 mm overlap) | Size (length x width) |
| 1 | Test pad | 20 mm x 4 mm |
| 2 | Absorption pad | 18 mm x 4 mm |
| 3 | Conjugation pad | 5 mm x 4 mm |
| 4 | Sample pad | 10 mm x 4 mm |
| 5 | Total | 53 mm x 4 mm |

**EXAMPLE 6: Antigen Detection Using Standard Samples of KLK2, IGFI, and PKCα**

To determine the limit of detection (LOD), cancer target antigens (IGFI, KLK2, PKCα) were prepared at various concentrations (1-8000 ng/mL) in PBS buffer (pH 7.4). A total sample volume of 100 μL was applied to the prepared LFA strips.

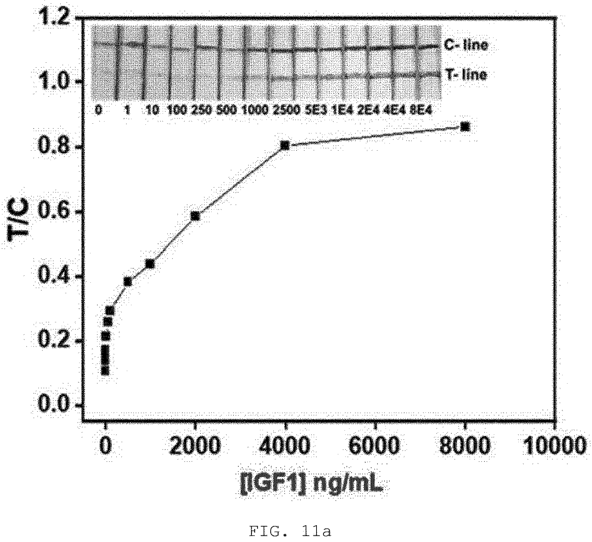

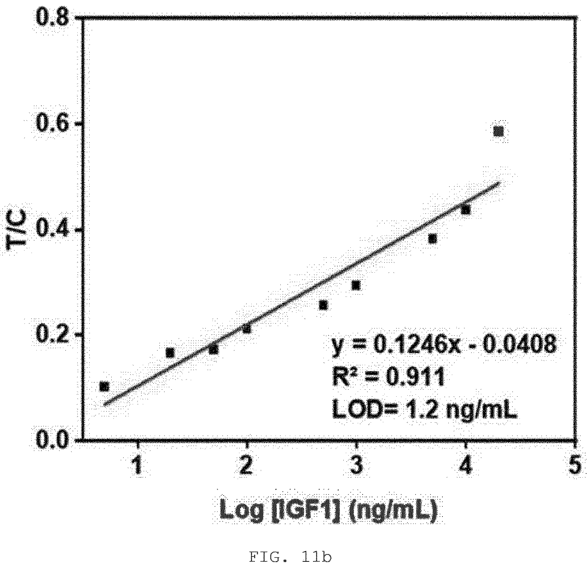

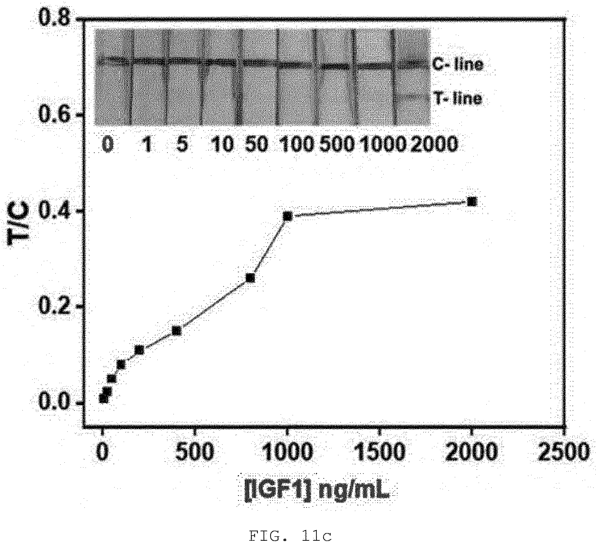

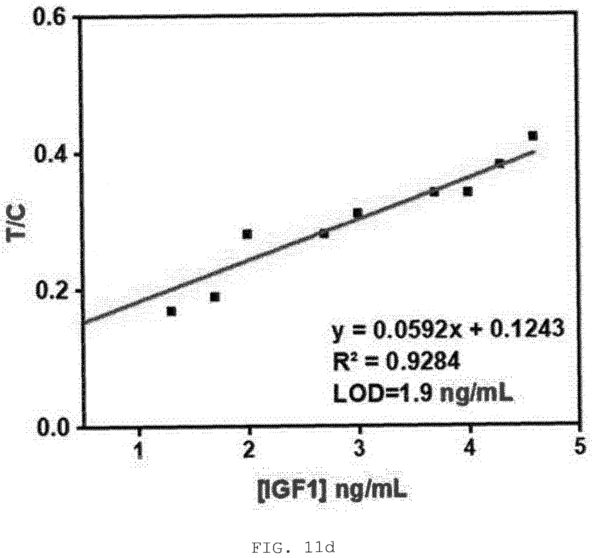

Detection results are given of IGFI in FIGS. 11a through 11d, KLK2 in FIGS. 12a to 12d, and PKCα in FIGS. 13a to 13d. As shown in the figures, recombinant antigens IGFI, KLK2, and PKCα were clearly detected at the T-line across various concentrations.

As the concentration of the target antigen decreased from 10,000 ng/mL to 1 ng/mL, a gradual decrease in the intensity of the TL color was observed. Conversely, as the antigen concentration increased, the red intensity of the TL deepened as expected. The control line (CL) remained consistently red regardless of the presence or absence of cancer biomarkers. At concentrations ranging from 1 or 10 ng/mL to 10,000 ng/mL, the TL color became thicker and more pronounced as the antigen concentration increased. LOD values for IGFI, KLK2, and PKCα antigens were measured as approximately 10 ng/mL, 1 ng/mL, and 50 ng/mL, respectively. The visual LOD for all target antigens on the TL was approximately 10 ng/mL.

Quantitative analysis of IGFI, KLK2, and PKCα was conducted by monitoring the T/C band intensities for the test and control lines. The band intensity gradually increased with increasing concentrations of IGFI, KLK2, and PKCα antigens. Based on these results, calibration curves for IGFI, KLK2, and PKCα were generated.

For IGFI, as shown in FIGS. 11a through 11d, good linearity was found between TL band intensity ($R^2$=0.911 and 0.928), and buffer and serum spike samples exhibited LOD values of as low as 1.2 ng/mL and 0.9 ng/mL, respectively.

As shown in FIGS. 12a through 12d, KLK2 showed good linearity between TL band intensities ($R^2$=0.969 and 0.977) and had buffer/serum spike samples with LOD values of as low as 2.1 ng/mL and 1.9 ng/mL, respectively.

As shown in FIGS. 13a through 13d, PKCα showed good linearity between TL band intensities ($R^2$=0.958 and 0.933), and buffer/serum spike samples with LOD values of as low as 1.5 ng/mL and 2.6 ng/mL, respectively.

Subsequently, ten test strips were randomly selected and evaluated for reaction times for LFA activity and line color intensity. From the measurements, it was concluded that the average reaction time was approximately 30 seconds, with maximum color intensity reached in 9.26 ± 1.58 minutes under PBS (pH 7.4) conditions. Thus, the total reaction time for the assay was considered to be under 10 minutes.

**EXAMPLE 7: Clinical Sample Analysis for Detection of IGFI, KLK2, and PKCα**

To evaluate the clinical utility of the LFA strips developed in the present disclosure, lateral flow assays were performed using actual blood serum samples from cancer patients. Specifically, LFA was conducted on 20 clinical samples (15 positive, 5 negative) using IGFI, KLK2, and PKCα antibody-based LFA strips, and the results were analyzed qualitatively and quantitatively. For titer measurements, clinical samples were diluted 1:20. The clinical samples included six categories: normal individuals, liver cancer, lung cancer, colorectal cancer, breast cancer, and prostate cancer.

Sensitivity, specificity, and accuracy were calculated using the following equations:

$$Sensitivity = \frac{true\ positive}{(true\ positive + false\ negative)} \; x \; 100$$

$$Specificity = \frac{true\ positive}{(true\ positive + false\ negative)} \; x \; 100$$

$$Accuracy = \frac{(true\ positive + true\ negative)}{total\ number\ of\ samples} \; x \; 100$$

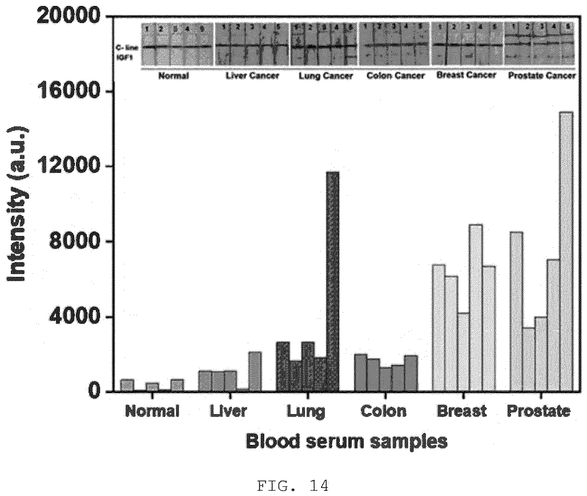

The LFA strips successfully detected IGFI, KLK2, and PKC$\alpha$ biomarkers in samples from patients with liver, lung, colorectal, breast, or prostate cancer (FIGS. 14 to 16).

Specifically, as shown in FIG. 14, IGFI was slightly detectable in normal samples but at significantly lower levels compared to cancer patient samples. High intensity of the test line (TL) was observed particularly in lung cancer, breast cancer, and prostate cancer samples, indicating its potential as a universal biomarker. The specificity, selectivity, and accuracy of the IGFI biomarker were approximately 96%, 92, and 87%, respectively.

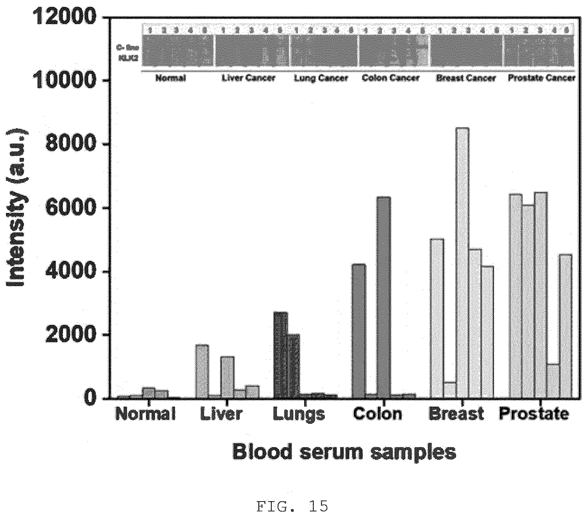

As shown in Figure 15, the KLK2 biomarker was analyzed using LFA strips across various cancer and normal samples. Generally, KLK2 exhibited significantly higher band intensity in cancer samples compared to normal samples. Notably, the TL showed a markedly stronger color intensity in breast cancer and prostate cancer samples compared to other cancer samples, demonstrating high specificity for breast cancer diagnosis. The sensitivity, selectivity, and accuracy of the KLK2 biomarker were approximately 100%, 100%, and 90%, respectively.

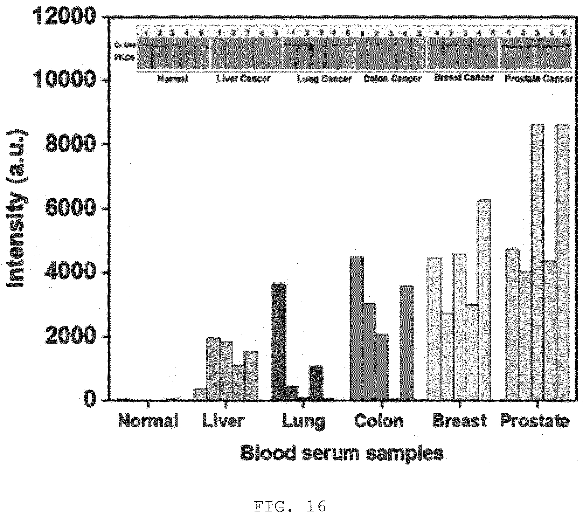

Similarly, as depicted in FIG. 16, the PKC$\alpha$ biomarker was analyzed using LFA strips across five cancer types and normal samples. Like the previously discussed biomarkers, PKC$\alpha$ also displayed higher band intensity in cancer samples compared to normal samples. In particular, rectal cancer, breast cancer, and prostate cancer samples exhibited notably higher color intensity on the test line (TL), confirming strong specificity for breast cancer and rectal cancer diagnosis. The sensitivity, selectivity, and accuracy of the PKC$\alpha$ biomarker were approximately 100%, 100%, and 80%, respectively.

**EXAMPLE 8: Lateral Flow Analysis Using LFA Strips for Multiple Biomarker Detection**

The preceding Examples validated the utility of each biomarker in the present disclosure as an effective diagnostic marker for cancer. To further enhance the utility of these biomarkers as diagnostic markers, the simultaneous detection of these biomarkers was investigated to determine whether it enables more accurate cancer diagnosis. Accordingly, an LFA strip capable of multiplex detection of PKC$\alpha$, IGFI, and KLK2 was prepared, with specific antibodies for all three biomarkers immobilized on the strip. Using this LFA strip, lateral flow analysis was performed. Likewise, the antibodies were conjugated with gold nanoparticles, and the sensitivity of the gold nanoparticle-based multiplex LFA was evaluated by detecting IGFI, KLK2, and PKC$\alpha$ spiked in PBS buffer at a concentration of 500 ng/mL.

FIGS. 17 through 23 illustrate the detection results of biomarkers in various samples using the gold nanoparticle-based multiplex detection LFA. FIGS. 17 and 18 display the results of spiked samples and normal samples, respectively, while FIGS. 19 through 23 show the results for cancer patient samples (in order: liver cancer, lung cancer, colorectal cancer, breast cancer, and prostate cancer). Each figure presents images of the test lines (TLs) for the three biomarkers (TL1 for PKC$\alpha$, TL2 for IGFI, and TL3 for KLK2) along with quantitative color intensity graphs. As observed in the figures, the color intensity of the TLs for normal serum samples was notably lower compared to those for serum samples from breast cancer, lung cancer, liver cancer, and colorectal cancer patients. When the multiplex detection LFA strip was applied to cancer patient samples, including those from breast cancer patients, the reddish coloration of gold nanoparticles was distinctly visible on the TLs. This indicates that the cancer patient samples contained high concentrations of IGFI, KLK2, and PKC$\alpha$ biomarkers.

Furthermore, these results collectively suggest that the gold nanoparticle-based multiplex detection LFA described in the present disclosure enables the simultaneous detection of biomarkers, thereby facilitating more accurate cancer diagnosis.

The results of the clinical sample analysis are summarized in Tables 6 and 7. These tables present cancer diagnostic outcomes obtained from clinical samples of cancer patients using enzyme-linked immunosorbent assay (ELISA) and lateral flow assay (LFA) for individual biomarkers. Positive rates are denoted as "+" and negative rates as "-". As shown in Table 6, when biomarkers were individually detected using ELISA, the negative rate was higher, leading to relatively lower diagnostic accuracy for cancer. In contrast, as observed in Table 7, simultaneous detection of biomarkers using the LFA strip described in the present disclosure significantly increased the positive diagnostic rate, enabling more accurate cancer diagnosis.

[TABLE 6]

| Cancer diagnosis results of individual biomarker detection in clinical samples from cancer patients by enzyme linked immunosorbent assay (ELISA) | | | | | |
|---|---|---|---|---|---|
| Biomarker | Breast Cancer | Prostate Cancer | Colorectal Cancer | Lung Cancer | Liver Cancer |
| PKCα | ++++ | +++ | - | - | - |
| IGF1 | + | +++ | - | - | - |
| KLK2 | + | +++ | - | - | - |
| 3G2 | +++ | ++++ | ++++ | ++ | +++ |
| 8B2 | ++ | +++ | +++ | + | ++ |
| 2D5 | ++ | ++ | +++ | - | ++ |

[TABLE 7]

| Cancer diagnosis results by lateral flow assay using the multi-biomarker LFA strip in samples from cancer patients | | | | | |
|---|---|---|---|---|---|
| Biomarker | Breast Cancer | Prostate Cancer | Colorectal Cancer | Lung Cancer | Liver Cancer |
| PKCα | +++ | ++ | +++ | ++ | +++++ |
| IGF1 | ++++ | +++ | +++++ | +++++ | +++++ |
| KLK2 | +++ | ++ | - | ++ | - |

These results indicate that the biomarkers used exhibit high activity in clinical samples from cancer patients and can be applied for the diagnosis of interrelated cancers such as breast cancer, prostate cancer, liver cancer, rectal cancer, lung cancer, and colorectal cancer.

It should be understood by those of ordinary skill in the art that the above description of the present disclosure is exemplary, and the exemplary embodiments disclosed herein can be easily modified into other specific forms without departing from the technical spirit or essential features of the present disclosure. Therefore, the exemplary embodiments described above should be interpreted as illustrative and not limited in any aspect.

Industrial Applicability

present disclosure pertains to multiple biomarkers for cancer diagnosis and uses thereof and, specifically, to diagnostic compositions comprising agents for measuring the protein or mRNA expression levels of IGFI, KLK2, PKCα, and TRPM8. The present inventors confirmed that the unique marker combination of IGFI, KLK2, PKCα, and TRPM8 in the present disclosure allows for rapid and accurate cancer diagnosis through the detection of these markers in actual cancer patient serum samples. Moreover, this approach enables the simultaneous detection of one or more cancers. Therefore, it is anticipated that the combination of IGFI, KLK2, PKCα, and TRPM8 can be used to diagnose various cancers, including prostate cancer, quickly and accurately. Consequently, the present disclosure has significant industrial applicability.

**Claims**

1. A cancer diagnostic composition comprising an agent that measures an expression level of at least one protein or mRNA selected from the group consisting of PKCα, IGFI, KLK2, and TRPM8.

2. The cancer diagnostic composition of claim 1, wherein the cancer diagnostic composition simultaneously detects one or more cancers.

3. The cancer diagnostic composition of claim 1, wherein the cancer is at least one selected from the group consisting of colorectal cancer, colon cancer, lung cancer, liver cancer, stomach cancer, esophageal cancer, pancreatic cancer, gallbladder cancer, kidney cancer, bladder cancer, prostate cancer, testicular cancer, cervical cancer, endometrial cancer, choriocarcinoma, ovarian cancer, breast cancer, thyroid cancer, brain cancer, head and neck cancer, malignant melanoma, lymphoma, aplastic anemia, and blood cancer.

4. The cancer diagnostic composition of claim 1, wherein the composition analyzes a sample selected from the group consisting of blood, plasma, serum, saliva, nasal fluid, sputum, synovial fluid, amniotic fluid, ascites, cervical or vaginal secretions, urine, and cerebrospinal fluid.

5. The cancer diagnostic composition of claim 1, wherein the protein is measured for expression level using an antibody or aptamer specific for the protein.

6. The cancer diagnostic composition of claim 1, wherein the mRNA is measured for expression level using a probe or primer binding specifically to the mRNA.

7. The cancer diagnostic composition of claim 1, wherein the composition further comprises an agent for measuring an expression level of a prostate-specific antigen (PSA) protein or an mRNA thereof.

8. A cancer diagnostic kit comprising the composition of claim 1.

9. The cancer diagnostic kit of claim 8, wherein the kit comprises a lateral flow assay strip.

10. The cancer diagnostic kit of claim 9, wherein the lateral flow assay strip comprises a sample pad, conjugation pad, a membrane, and an absorbent pad.

11. The cancer diagnostic kit of claim 9, wherein the lateral flow assay strip comprises gold nanoparticles conjugated with at least one antibody selected from the group consisting of an anti-PKC$\alpha$ antibody, an anti-IGFI antibody, an anti-KLK2 antibody, and an anti-TRPM8 antibody.

12. A method for providing information for cancer diagnosis, the method comprising the steps of:

   (a) measuring an expression level of at least one protein or mRNA selected from the group consisting of PKC$\alpha$, IGFI, KLK2, and TRPM8 in a biological sample isolated from a subject; and
   (b) diagnosing cancer or determining cancer risk when the measured expression level of the protein or mRNA is increased compared to a control group.

13. The method of claim 12, wherein the expression level of the protein is measured using enzyme-linked immunosorbent assay (ELISA) or lateral flow assay.

**FIG. 1a**

**FIG. 1b**

FIG. 1c

pH: Control 4  5  6  7  8  9 10

1 day

pH: Control 4  5  6  7  8  9 10

7 days

FIG. 1d

FIG. 1e

FIG. 1f

FIG. 1g

FIG. 1h

FIG. 1i

FIG. 1j

FIG. 1k

FIG. 11

FIG. 2

EP 4 571 314 A1

32

FIG. 3

FIG. 4

FIG. 5

FIG. 6

FIG. 7

FIG. 8

FIG. 9

FIG. 10a

FIG. 10b

FIG. 10c

FIG. 10d

FIG. 10e

FIG. 10f

FIG. 11a

FIG. 11b

FIG. 11c

FIG. 11d

FIG. 12a

y = 0.1379x + 0.1515
R² = 0.9694
LOD= 2.1 ng/mL

FIG. 12b

FIG. 12c

FIG. 12d

FIG. 13a

FIG. 13b

FIG. 13c

FIG. 13d

FIG. 14

FIG. 15

FIG. 16

FIG. 17

FIG. 18

FIG. 19

FIG. 20

FIG. 21

FIG. 22

FIG. 23

## INTERNATIONAL SEARCH REPORT

| International application No. |
|---|
| **PCT/KR2023/011992** |

| A. | CLASSIFICATION OF SUBJECT MATTER |
|---|---|

G01N 33/574(2006.01)i; G01N 33/58(2006.01)i; G01N 33/543(2006.01)i; C12Q 1/6886(2018.01)i

According to International Patent Classification (IPC) or to both national classification and IPC

| B. | FIELDS SEARCHED |
|---|---|

Minimum documentation searched (classification system followed by classification symbols)

G01N 33/574(2006.01); C12Q 1/68(2006.01); G06F 19/00(2011.01); G16B 20/00(2019.01); G16B 40/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: PKCα, IGF1(Insulin-like growth factor 1), KLK2(human Kallikrein), TRPM8, 암 (cancer), 바이오마커(biomarker)

| C. | DOCUMENTS CONSIDERED TO BE RELEVANT |
|---|---|

| Category* | Citation of document, with indication, where appropriate, of the relevant passages | Relevant to claim No. |
|---|---|---|
| X<br>A | AIDOO-BROWN, J. et al. Multiplexed Prostate Cancer Companion Diagnostic Devices. Sensors. 2021, vol. 21, thesis no. 5023, inner pp. 1-33 (publication date: 24 July 2021).<br>See abstract, pages 8 and 15, Table 1 and Figure 9. | 1,3-13<br><br>2 |
| X | KR 10-2013-0054311 A (BIOINFRA INC. et al.) 24 May 2013 (2013-05-24)<br>See abstract, claim 1, paragraphs [0016]-[0026] and [0187] and tables 5-7. | 1-8 |
| A | WO 2016-145308 A2 (JANSSEN PHARMACEUTICA NV) 15 September 2016 (2016-09-15)<br>See claim 26. | 1-13 |
| A | KANG, Jeong-Hun et al. Plasma protein kinase C (PKC)α as a biomarker for the diagnosis of cancers. Carcinogenesis. 2009, vol. 30, no. 11, pp. 1927–1931 (publication date: 26 August 2009).<br>See abstract. | 1-13 |
| A | KR 10-2017-0008729 A (OPKO DIAGNOSTICS, LLC) 24 January 2017 (2017-01-24)<br>See paragraph [0004]. | 1-13 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
|---|---|
| **16 November 2023** | **16 November 2023** |

| Name and mailing address of the ISA/KR | Authorized officer |
|---|---|
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2023/011992**

| Patent document cited in search report | | | Publication date (day/month/year) | Patent family member(s) | | | Publication date (day/month/year) |
|---|---|---|---|---|---|---|---|
| KR | 10-2013-0054311 | A | 24 May 2013 | KR | 10-1401561 | B1 | 11 June 2014 |
| | | | | KR | 10-1454398 | B1 | 13 November 2014 |
| | | | | KR | 10-2012-0077567 | A | 10 July 2012 |
| | | | | KR | 10-2012-0077570 | A | 10 July 2012 |
| | | | | KR | 10-2012-0134091 | A | 11 December 2012 |
| | | | | KR | 10-2012-0134092 | A | 11 December 2012 |
| | | | | KR | 10-2013-0004203 | A | 09 January 2013 |
| | | | | KR | 10-2013-0004204 | A | 09 January 2013 |
| | | | | WO | 2012-091506 | A2 | 05 July 2012 |
| | | | | WO | 2012-091506 | A3 | 24 January 2013 |
| WO | 2016-145308 | A2 | 15 September 2016 | CA | 2979226 | A1 | 15 September 2016 |
| | | | | CN | 107567502 | A | 09 January 2018 |
| | | | | CY | 1125020 | T1 | 24 March 2023 |
| | | | | DK | 3268493 | T3 | 07 March 2022 |
| | | | | EP | 3268493 | A2 | 17 January 2018 |
| | | | | EP | 3268493 | B1 | 05 January 2022 |
| | | | | ES | 2905163 | T3 | 07 April 2022 |
| | | | | HR | P20220128 | T1 | 29 April 2022 |
| | | | | HU | E058025 | T2 | 28 June 2022 |
| | | | | IL | 254119 | A | 31 October 2017 |
| | | | | IL | 254119 | D0 | 31 October 2017 |
| | | | | JP | 2018-507702 | A | 22 March 2018 |
| | | | | KR | 10-2017-0136525 | A | 11 December 2017 |
| | | | | LT | 3268493 | T | 10 March 2022 |
| | | | | MA | 45480 | A | 17 January 2018 |
| | | | | PL | 3268493 | T3 | 09 May 2022 |
| | | | | PT | 3268493 | T | 13 April 2022 |
| | | | | RS | 62972 | B1 | 31 March 2022 |
| | | | | SI | 3268493 | T1 | 29 April 2022 |
| | | | | US | 2016-0304962 | A1 | 20 October 2016 |
| | | | | WO | 2016-145308 | A3 | 13 October 2016 |
| KR | 10-2017-0008729 | A | 24 January 2017 | AR | 099883 | A1 | 24 August 2016 |
| | | | | AU | 2015-237270 | A1 | 01 October 2015 |
| | | | | AU | 2015-237270 | A1 | 20 October 2016 |
| | | | | AU | 2015-237270 | B2 | 09 September 2021 |
| | | | | AU | 2016-254127 | A1 | 26 October 2017 |
| | | | | AU | 2016-254127 | B2 | 26 May 2022 |
| | | | | BR | 112016022407 | A2 | 12 December 2017 |
| | | | | BR | 112017022391 | A2 | 10 July 2018 |
| | | | | CA | 2944001 | A1 | 01 October 2015 |
| | | | | CA | 2944001 | C | 15 August 2023 |
| | | | | CA | 2982262 | A1 | 03 November 2016 |
| | | | | CL | 2016002419 | A1 | 31 March 2017 |
| | | | | CL | 2017002701 | A1 | 06 July 2018 |
| | | | | CN | 106663149 | A | 10 May 2017 |
| | | | | CN | 107530412 | A | 02 January 2018 |
| | | | | CN | 114740202 | A | 12 July 2022 |
| | | | | CO | 2017010808 | A2 | 19 April 2018 |
| | | | | DE | 202015009668 | U1 | 21 January 2019 |
| | | | | DE | 202016008673 | U1 | 22 January 2019 |

Form PCT/ISA/210 (patent family annex) (July 2022)

<table>
<tr><td colspan="2"><b>INTERNATIONAL SEARCH REPORT</b><br>Information on patent family members</td><td colspan="2">International application No.<br><b>PCT/KR2023/011992</b></td></tr>
<tr><td>Patent document<br>cited in search report</td><td>Publication date<br>(day/month/year)</td><td>Patent family member(s)</td><td>Publication date<br>(day/month/year)</td></tr>
<tr><td></td><td></td><td>EA 039028 B1</td><td>24 November 2021</td></tr>
<tr><td></td><td></td><td>EA 201691952 A1</td><td>31 May 2017</td></tr>
<tr><td></td><td></td><td>EA 201792372 A1</td><td>30 March 2018</td></tr>
<tr><td></td><td></td><td>EP 3123381 A1</td><td>01 February 2017</td></tr>
<tr><td></td><td></td><td>EP 3123381 B1</td><td>11 October 2023</td></tr>
<tr><td></td><td></td><td>EP 3247387 A1</td><td>29 November 2017</td></tr>
<tr><td></td><td></td><td>EP 3247387 A4</td><td>12 September 2018</td></tr>
<tr><td></td><td></td><td>EP 3287786 A2</td><td>28 February 2018</td></tr>
<tr><td></td><td></td><td>EP 3287786 A3</td><td>21 March 2018</td></tr>
<tr><td></td><td></td><td>EP 3299977 A1</td><td>28 March 2018</td></tr>
<tr><td></td><td></td><td>HK 1247559 A1</td><td>28 September 2018</td></tr>
<tr><td></td><td></td><td>HK 1247824 A1</td><td>05 October 2018</td></tr>
<tr><td></td><td></td><td>IL 247981 A</td><td>30 November 2016</td></tr>
<tr><td></td><td></td><td>IL 247981 B</td><td>28 February 2021</td></tr>
<tr><td></td><td></td><td>IL 247981 D0</td><td>30 November 2016</td></tr>
<tr><td></td><td></td><td>IL 254833 A0</td><td>31 December 2017</td></tr>
<tr><td></td><td></td><td>IL 254833 B</td><td>01 December 2021</td></tr>
<tr><td></td><td></td><td>IL 254833 D0</td><td>31 December 2017</td></tr>
<tr><td></td><td></td><td>IL 280039 A</td><td>01 March 2021</td></tr>
<tr><td></td><td></td><td>IL 280039 B</td><td>01 December 2021</td></tr>
<tr><td></td><td></td><td>IL 280039 D0</td><td>01 March 2021</td></tr>
<tr><td></td><td></td><td>JP 2017-515127 A</td><td>08 June 2017</td></tr>
<tr><td></td><td></td><td>JP 2018-518661 A</td><td>12 July 2018</td></tr>
<tr><td></td><td></td><td>JP 2021-009157 A</td><td>28 January 2021</td></tr>
<tr><td></td><td></td><td>JP 2023-017841 A</td><td>07 February 2023</td></tr>
<tr><td></td><td></td><td>JP 6775488 B2</td><td>28 October 2020</td></tr>
<tr><td></td><td></td><td>JP 6873916 B2</td><td>19 May 2021</td></tr>
<tr><td></td><td></td><td>JP 7256781 B2</td><td>12 April 2023</td></tr>
<tr><td></td><td></td><td>KR 20180018514 A</td><td>21 February 2018</td></tr>
<tr><td></td><td></td><td>MX 2016012667 A</td><td>09 January 2017</td></tr>
<tr><td></td><td></td><td>MX 2017013881 A</td><td>12 March 2018</td></tr>
<tr><td></td><td></td><td>MX 2022002365 A</td><td>06 April 2022</td></tr>
<tr><td></td><td></td><td>MY 192513 A</td><td>24 August 2022</td></tr>
<tr><td></td><td></td><td>PE 02982017 A1</td><td>18 April 2017</td></tr>
<tr><td></td><td></td><td>PE 20170298 A1</td><td>18 April 2017</td></tr>
<tr><td></td><td></td><td>PE 20180930 A1</td><td>08 June 2018</td></tr>
<tr><td></td><td></td><td>PE 9302018 A1</td><td>08 June 2018</td></tr>
<tr><td></td><td></td><td>SG 10201808585 A</td><td>29 November 2018</td></tr>
<tr><td></td><td></td><td>SG 11201608035 A</td><td>28 October 2016</td></tr>
<tr><td></td><td></td><td>SG 11201708289 A</td><td>29 November 2017</td></tr>
<tr><td></td><td></td><td>TW 201621320 A</td><td>16 June 2016</td></tr>
<tr><td></td><td></td><td>TW 201705028 A</td><td>01 February 2017</td></tr>
<tr><td></td><td></td><td>TW I687688 B</td><td>11 March 2020</td></tr>
<tr><td></td><td></td><td>TW I766836 B</td><td>11 June 2022</td></tr>
<tr><td></td><td></td><td>UA 125745 C2</td><td>01 June 2022</td></tr>
<tr><td></td><td></td><td>US 11761962 B2</td><td>19 September 2023</td></tr>
<tr><td></td><td></td><td>US 2016-0025732 A1</td><td>28 January 2016</td></tr>
<tr><td></td><td></td><td>US 2016-320394 A1</td><td>03 November 2016</td></tr>
<tr><td></td><td></td><td>US 2017-089904 A1</td><td>30 March 2017</td></tr>
<tr><td></td><td></td><td>US 2022-399121 A1</td><td>15 December 2022</td></tr>
</table>

Form PCT/ISA/210 (patent family annex) (July 2022)

**INTERNATIONAL SEARCH REPORT**
Information on patent family members

International application No.

**PCT/KR2023/011992**

| Patent document cited in search report | Publication date (day/month/year) | Patent family member(s) | | Publication date (day/month/year) |
|---|---|---|---|---|
| | | WO 2015-148979 | A1 | 01 October 2015 |
| | | WO 2015-148979 | A8 | 07 January 2016 |
| | | WO 2016-176529 | A1 | 03 November 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220101571

**Non-patent literature cited in the description**

- **HARLOW** ; **LANE**. Antibodies: A Laboratory Manual. Cold Spring Harbor, 1988
- **BRABANT**. *Eur. J. Endocrinol.*, 2003, vol. 148, S15-S20
- **GLYNN** ; **AGHA**. *Inter. J. Endocrinol*, 2012, vol. 972627, 2012
- **YU et al.** *J. Natl. Cancer Inst.*, 1999, vol. 91, 151-156
- **LI et al.** *Cell. Phys. Biochem.*, 2016, vol. 38, 589-597
- **LEROITH** ; **ROBERTS**. *Cancer Lett.*, 2003, vol. 195, 127-137
- **CHAN et al.** *Science*, 1998, vol. 279, 563-566
- **POLLAK et al.** *Nat. Rev. Cancer*, 2004, vol. 4, 505-518
- **VELLA et al.** *Biochim. Biophys. Acta, Mol. Cell Res*, vol. 1866, 118522
- **WANG et al.** *Oncogene*, 2019, vol. 38, 6338-6353
- **JANICZEK et al.** *J. Immunol. Res.*, 2020, vol. 4910595, 2020
- **AHEARN et al.** *Carcinogenesis*, 2018, vol. 39, 1431-1437
- **KOLCZYNSKA et al.** *Lipids Health Dis*, 2020, vol. 19, 1-15
- **GRINER** ; **KAZANIETZ**. *Nat. Rev. Cancer*, 2007, vol. 7, 281-294
- **HANAUSKE et al.** *Curr. Pharm. Des.*, 2004, vol. 10, 1923-1936
- **GARZOTTO et al.** *Cancer Res.*, 1999, vol. 59, 5194-5201
- **DEVERAUX et al.** *EMBO J.*, 1998, vol. 17, 2215-2223
- **ZIMMERMANN** ; **GREEN**. *J. Allergy Clin. Immunol.*, 2001, vol. 108, S99-S103
- **AGGARWAL**. *Nat. Rev. Immunol.*, 2003, vol. 3, 745-756
- **ISAKOV, N.** Protein kinase C (PKC) isoforms in cancer, tumor promotion and tumor suppression. *Seminars in cancer biology*, 2018
- **MASUR et al.** *Mol. Biol. Cell*, 2001, vol. 12, 1973-1982
- **GARRETT** ; **SELL**. Cellular cancer markers.. Springer Science & Business Media, 2013, vol. 12
- **GRETZER** ; **PARTIN**. *Urol. Clin. North Am.*, 2003, vol. 30, 677-686
- **ANDERIESZ** ; **QUINN**. *Med, J. Aust.*, 2003, vol. 178, 655-656
- **DUFFY et al.** *Tumor Biol.*, 2018, vol. 40, 1010428318776169